# EUROPEAN PATENT SPECIFICATION

(11) **EP 2 350 668 B1**
(45) Date of publication and mention of the grant of the patent: **20.06.2012**
(21) Application number: 09765778.7
(22) Date of filing: 10.06.2009
(51) Int. Cl.: G01N 33/68, G01N 33/50

(54) **DRUG SAFETY TEST USING PENTRAXIN-3 (PTX-3)**
ARZNEISTOFFSICHERHEITSTEST MIT PENTRAXIN-3 (PTX-3)
TEST D'INNOCUITÉ DE MÉDICAMENT À L'AIDE DE LA PENTRAXINE-3 (PTX-3)

(30) Priority: 20.06.2008 EP 08158724
(43) Date of publication of application: 03.08.2011
(73) Proprietor: F. Hoffmann-La Roche AG, 4070 Basel (CH)
(72) Inventor: KROPSHOFER, Harald, 79539 Loerrach (DE); PLOIX, Corinne, F-68400 Riedisheim (FR); VOGT, Anne, 79539 Loerrach (DE)
(74) Representative: Küng, Peter
(86) International application number: PCT/EP2009/057146
(87) International publication number: WO 2009/153198

(56) References cited:
- SADORGE ET AL: "Phase 1 clinical trial of live attenuated Shigella dysenteriae type-1 DELTAicsA DELTAent DELTAfep DELTAstxA:HgR oral vaccine SC599 in healthy human adult volunteers" VACCINE, BUTTERWORTH SCIENTIFIC. GUILDFORD, GB, vol. 26, no. 7, 3 December 2007 (2007-12-03), pages 978-987, XP022438263 ISSN: 0264-410X
- WING M G ET AL: "Ex-vivo whole blood cultures for predicting cytokine-release syndrome: dependence on target antigen and antibody isotype" 1 August 1995 (1995-08-01), 19950801 , XP002539428 abstract
- GARLANDA CECILIA ET AL: "Pentraxins at the crossroads between innate immunity, inflammation, matrix deposition, and female fertility." 2005, ANNUAL REVIEW OF IMMUNOLOGY 2005, VOL. 23, PAGE(S) 337 - 366 , XP002539429 ISSN: 0732-0582 page 338 page 349 page 354 - page 358
- AZZURRI A ET AL: "IFN-gamma-inducible protein 10 and pentraxin 3 plasma levels are tools for monitoring inflammation and disease activity in Mycobacterium tuberculosis infection" MICROBES AND INFECTION, ELSEVIER, PARIS, FR, vol. 7, no. 1, 1 January 2005 (2005-01-01), pages 1-8, XP004742531 ISSN: 1286-4579
- SUZUKI ET AL: "Pentraxin 3, a new marker for vascular inflammation, predicts adverse clinical outcomes in patients with heart failure" AMERICAN HEART JOURNAL, MOSBY- YEAR BOOK INC., ST. LOUIS, MO, US, vol. 155, no. 1, 1 January 2008 (2008-01-01), pages 75-81, XP022408102 ISSN: 0002-8703
- PERI G ET AL: "PTX3, A prototypical long pentraxin, is an early indicator of acute myocardial infarction in humans." 8 August 2000 (2000-08-08), CIRCULATION 8 AUG 2000, VOL. 102, NR. 6, PAGE(S) 636 - 641 , XP002539430 ISSN: 1524-4539 the whole document
- SULIMAN M E ET AL: "The long pentraxin PTX-3 in prevalent hemodialysis patients: associations with comorbidities and mortality." May 2008 (2008-05), QJM : MONTHLY JOURNAL OF THE ASSOCIATION OF PHYSICIANS MAY 2008, VOL. 101, NR. 5, PAGE(S) 397 - 405 , XP002539431 ISSN: 1460-2393 the whole document
- BOTTAZZI B ET AL: "Pentraxins as a key component of innate immunity" CURRENT OPINION IN IMMUNOLOGY, ELSEVIER, OXFORD, GB, vol. 18, no. 1, 1 February 2006 (2006-02-01), pages 10-15, XP025078963 ISSN: 0952-7915 [retrieved on 2006-02-01]
- MULLER B ET AL: "CIRCULATING LEVELS OF THE LONG PENTRAXIN PTX3 CORRELATE WITH SEVERITY OF INFECTION IN CRITICALLY ILL PATIENTS" CRITICAL CARE MEDICINE, WILLIAMS AND WILKINGS COMPANY, BALTIMORE, MA, US, vol. 29, no. 7, 1 July 2001 (2001-07-01), pages 1404-1407, XP009060510 ISSN: 0090-3493

## Description

In the past, administration of therapeutic antibodies has caused serious side-effects related to the first infusion of said antibodies during clinical studies. Acute release of excessive amounts of particular pro-inflammatory cytokines in the circulation has been described and is thought to be critical in causing a complex of symptoms, denoted as cytokine release syndrome (CRS). Usually CRS starts 20-90 min post-infusion and is the result of the release of cytokines from cells targeted by the antibody as well as from immune effector cells triggered via the binding of the antibody to Fc receptors. When cytokines are released into the circulation, systemic symptoms such as fever, nausea, chills, hypotension, tachycardia, asthenia, headache, rash, scratchy throat, and dyspnea can result. In most patients, the symptoms are mild to moderate in severity and are managed easily. However, some patients may experience severe, life-threatening reactions that may result from massive release of cytokines.

As the CRS is a species-specific event that cannot be observed in conventional animal models of drug testing, *in vitro* models based on human blood derived cells are needed to assess the risk of cytokine release of new medicinal products. Known are assays which measure cytokine levels in blood serum or plasma (WingMG et aL, Ther Immunol. 1995, 2(4): 183-190). However, the increase of classical cytokines such as TNF- α, IFN- γ or interleukins is not always predictive for CRS occurrences. Furthermore, with the available assays the cytokine release in blood samples is detectable only after a considerable amount of time after a potential adverse event. Thus, there is a need to provide a reliable and faster method for determining the risk of a compound to induce CRS. This problem is solved by the present invention, in particular by the use of Pentraxin-3 (PTX-3) for determining the risk of a compound with respect to the induction of acute cytokine release-related infusion reactions.

The present invention provides a new method for determining the risk of a compound of interest to induce a CRS in an individual.

Said method comprises the steps of:
a) providing a whole blood sample, wherein said blood sample was exposed at least 30 minutes to said compound,
b) measuring the level of pentraxin-3 (PTX-3) in said sample, and
c) comparing the measured level of pentraxin-3 with a control, wherein a control is the pentraxin-3 level of an unexposed whole blook sample, wherein a significantly increased level of pentraxin-3 as compared to the control indicates a risk of said compound to induce a cytokine release syndrome.

Preferably, the PTX-3 level is measured in the plasma which is obtained by removing the blood cells from the whole blood sample. Alternatively, the PTX-3 level can also be measured in the serum obtained from the whole blood sample.

Immunogenicity refers to the ability of a substance to trigger an immune response. An immune response is a reaction of the immune system to substances, mediated by B-cells, T-cells, and cells of the innate immune system, such as monocytes, macrophages or neutrophilic granulocytes. A B-cell reaction involves the production and release of antigen-specific antibodies. Responses ofT cells, monocytes or granulocytes can involve the release of pro-inflammatory and/or anti-inflammatory cytokines. An acute release of excessive amounts of particular pro-inflammatory cytokines in the circulation of the body is denoted as "cytokine release syndrome" (CRS).

A cytokine is a soluble protein that acts as a messenger between cells, either stimulating or inhibiting the activity of various cells of the immune system. Cytokines include lymphokines, monokines, interleukins and interferons.

The term "compound of interest" as used herein refers to biological or chemical molecule. A biological molecule can be in particular a polypeptide or a polynucleotide. Said polypeptide and polynucleotide can have modifications, such as e.g. methylation, pegylation, phosphorylation and glycosylation.

The term "polypeptide" as used herein refers to a chain of amino acids connected by peptide bonds. The term "polypeptide" includes also a protein and a peptide. Preferably, a polypeptide is an antibody, more preferably, a therapeutic antibody. A therapeutic antibody can be a human or humanized antibody.

The term "polynucleotide" as used herein relates to a polymer of nucleotides comprising nucleotides A, C, G and T (or U). A polynucleotide is e.g. an oligonucleotide, a miRNA, a siRNA, an antisense RNA, mRNA or cDNA.

Preferably, a compound of interest is a therapeutic compound. A therapeutic compound is a compound for use in curative methods, including alleviating, removing or lessening symptoms of or preventing or reducing the possibility of contracting any disorder or malfunction of the human or animal body. A therapeutic compound can be a biological or a chemical molecule. A biological therapeutic compound is preferably an antibody or a siRNA.

Pentraxin-3 (PTX-3), also known as Pentaxin-3, belongs to a family of proteins known as 'long pentraxins', which are characterized by a carboxyterminal Pentraxin domain (Basile et al, J Biol Chem. 1997, 272(13):8172-8). The cDNA encoding this protein of 381 amino acids (SEQ. ID NO: 1) has been cloned by Breviario et al (J Biol Chem., 1992, 267(31): 22190-22197) and identified as a gene the expression of which is induced in human umbilical vein endothelial cells by IL-1β.

Pentraxin-3 is produced and secreted by a variety of cell types. Human peripheral blood mononuclear cells produce the protein in response to bacterial lipopolysaccharides, IL-1β, and TNF- α but not in response to IL-6, MCP-1, M-CSF, GM-CSF, or IFN-γ (Alles et al, Blood. 1994, 84(10):3483-93). Pentraxin is expressed in stimulated monocytes, endothelial cells, and fibroblasts (Alles et al, 1994), dendritic cells (Doni et al, Eur J Immunol. 2003, 33(10):2886-93), undifferentiated and differentiated myoblasts (Introna et al, Blood. 1996, 87(5):1862-72), rheumatoid arthritis synoviocytes (Luchetti et al, Clin Exp Immunol. 2000, 119(1):196-202.), glial cells (Polentarutti et al, J Neuroimmunol. 2000 Jul 1;106(1-2):87-94), Kaposi sarcoma cells (inducible by viral IL-6) (Klouche et al, AIDS. 2002 May 24;16(8):F9-18), macrophages and endothelial cells and infrequently by smooth muscle cells in advanced atherosclerotic plaques (Rolph et al, Arterioscler Thromb Vasc Biol. 2002; 22(5):e10-4), glomerular mesangial and endothelial cells (Bussolati et al, J Immunol. 2003 Feb 1; 170(3):1466-72), pre-adipocytes (Abderrahim-Ferkoune et al, J Lipid Res. 2003;44(5):994-1000), human and murine cumulus oophorus cells (Salustri et al, Development. 2004; 131(7):1577-86) and in neutrophils (Jaillon et al, JEM. 2007, 204(4):793-804; Imamura et al, Cell Immunol 2007, 248(2):86-94).

The term "whole blood" as used herein refers to a fluid which comprises red blood cells, white blood cells, and platelets suspended in a fluid called plasma. Whole blood samples can be collected from any animal. Preferably, the whole blood sample is collected from a human patient (human whole blood sample).

There are several methods for exposing whole blood to a compound of interest:
Method 1) One possibility is to add the compound to a whole blood sample. The compound is added as soon as possible after the sample has been collected, preferably within 4 hours after collection, more preferably 0.5 to 3 hours after collection. Most preferably, the compound is added within 30 to 60 minutes after the collection.

There is an incubation period between adding the compound to the whole blood sample and processing the sample. The incubation starts preferably within 4 hours after the collection of the blood sample. The whole blood sample is incubated preferably for at least 30 minutes with the compound of interest. More preferably, the incubation period is at least 1 hour, even more preferred is an incubation of the whole blood sample with the compound of interest for at least 2 hours. Preferably, the whole blood sample is incubated with the compound of interest for not more than 48 hours, more preferably, not more than 24 hours. A preferred incubation period is 30 minutes to 24 hours. A further preferred incubation period is 2h to 24 hours. More preferably, the incubation period is 30 minutes to 4 hours.

The incubation is done at room temperature (20°C) or higher. Preferably, the sample is incubated under physiological conditions (Temperature: 35-39°C, preferably about 37°C; CO₂ concentration of 3 to 7%, preferably about 5%).

Method 2) The second possibility is to administer the compound of interest to the donor (e.g. by infusion) and to collect a whole blood sample afterwards.

Between administering the compound of interest and collecting the whole blood sample is a waiting period. Said waiting period is between 30 minutes and 24 hours.

A combination of the two alternatives is possible, i.e. administering the compound of interest to the donor, collecting a whole blood sample of the donor, and adding the compound of interest to the whole blood sample.

After the exposure, the sample is further processed. Further processing includes but is not limited to removing the blood cells from the whole blood sample. Optionally, fibrinogen and clotting factors are additionally removed from the sample. Preferably, the whole blood sample is further processed within 4 hours after collection from the donor, more preferably within 3 hours after collection of the blood sample from the donor. Most preferably, the processing of the whole blood sample is done within 30 to 60 minutes after collection of the blood sample from the donor. In case of incubation of the whole blood sample with the compound of interest (exposure according to method 1 or a combination of the method 1 and 2) the further processing of the blood sample is done after the incubation period, preferably within 5 to 60 minutes after the end of the incubation time.

PTX-3 can be measured in the serum or plasma derived from the said whole blood sample. The measurements of the PTX-3 level may be performed in fresh plasma or serum which was obtained from the whole blood sample after the incubation with the compound of interest. Alternatively, the plasma or serum may be frozen and at a later date thawed to measure the PTX-3 level.

The term "plasma" refers to the liquid portion of the blood. Plasma is obtained by removing the blood cells (erythrocytes, leukocytes, thrombocytes) by centrifugation. The supernatant (= plasma) comprises serum, fibrinogen and other clotting factors. Serum is therefore plasma without clotting factors.

The term "blood cells" refers to is any cell of any type normally found in blood. Blood cells include but are not limited to Erythrocytes (red blood cell), Leukocytes (white blood cells), and Thrombocytes (platelets).

To prevent or delay coagulation of the whole blood sample or the plasma, an anticoagulant is added to the sample. Preferably, the anticoagulant is heparin (e.g. Na-heparin, e.g. 30USP to 143USP units of sodium Heparin (freeze dried) or 60 to 90 USP Units of sodium Heparin (spray-coated)), citrate (e.g. end concentration of about 0.4%. or oxalate. Usually the anticoagulant is coated on the inner surface of the container (e.g. tube) which contains the sample. Such coated tubes are well known to the skilled in the art (e.g. Vacutainer from BD Diagnostic Systems or vacuette from Greiner Bio-one). Another possibility is to add the anticoagulant to the sample within 5 to 15 minutes after collection (preferably less than 5 minutes after collection, more preferably less than 1 minute after collection) of the blood. Heparinated plasma is plasma which comprises heparin to prevent coagulation.

Methods for measuring PTX-3 are well known to the skilled person in the art. It may be measured for example by using an enzyme-linked immunosorbent assay (ELISA). ELISA tests are well known to the skilled person in the art. For measuring PTX-3 a sandwich ELISA is typically used. Kits are commercially available (e.g. Cat. No. ALX-850-299 from ALEXIS Biochemicals). Preferably, the PTX-3 level is measured with Enhanced Chemiluminescence (ECL) ELISA.

A sandwich ELISA is e.g. performed by (1) coating a solid support (e.g. a plate) with a capture antibody; (2) adding the sample (plasma), and any antigen present binds to the capture antibody; (3) adding a detecting antibody which binds to the antigen; (4) adding a enzyme-linked secondary antibody which binds to the detecting antibody; (5) adding a substrate, which is converted by the enzyme to a detectable form.

In an ECL ELSA, the enzyme linked to the secondary antibody converts directly or indirectly an ECL substrate to a form which emits chemiluminesence. A suitable enzyme is e.g. the horseradish peroxidase enzyme (HRP). A suitable ECL substrate is e.g. Luminal. The enzyme catalyzes the conversion of the ECL substrate into a sensitized reagent in the vicinity of the molecule of interest, which on further oxidation by hydrogen peroxide, produces a triplet (excited) carbonyl which emits light when it decays to the singlet carbonyl. Enhanced chemiluminescence allows detection of minute quantities of a biomolecule. Proteins can be detected down to femtomolar quantities.

Another method for measuring PTX3 is a colorimetry ELISA assay. In a colorimetric ELISA, the enzyme linked to the secondary antibody converts directly or indirectly a substrate to a form which is detectable via spectrophotometry. A suitable enzyme is the horseradish peroxidase enzyme (HRP) in combination with e.g. TMB (tetramethyl benzidine), DAB (3,3'-Diaminobenzidin), AEC (3-Amino-9-Ethylcarbazol), or CN (4-Chlor-1-Naphthol). Another suitable enzyme is the alkaline phosphatase (AP) in combination with e.g. Naphthol AS-MX phosphate or nitro-blue tetrazolium/5-brom-4-chlor-3-indoxylphosphate p-toluidine (NBT/BCIP).

A compound of interest has a risk to induce CRS in vivo if the level of measured PTX-3 after exposure to said compound is significantly increased compared to the PTX-3 level of a control.

A control is the pentraxin-3 level of an unexposed whole blood sample. Unexposed means that said blood was not exposed to the compound of interest nor to another compound known to trigger cytokine release under physiological conditions. Preferably, the blood was exposed to no substance at all before processing.

Significantly increased means that the level is higher compared to the control level and that the difference to the control level is statistically relevant (p ≤0.05, preferably, p ≤ 0.01).

Having now generally described this invention, the same will become better understood by reference to the specific examples, which are included herein for purpose of illustration only and are not intended to be limiting unless otherwise specified, in connection with the following figures.

### Figures

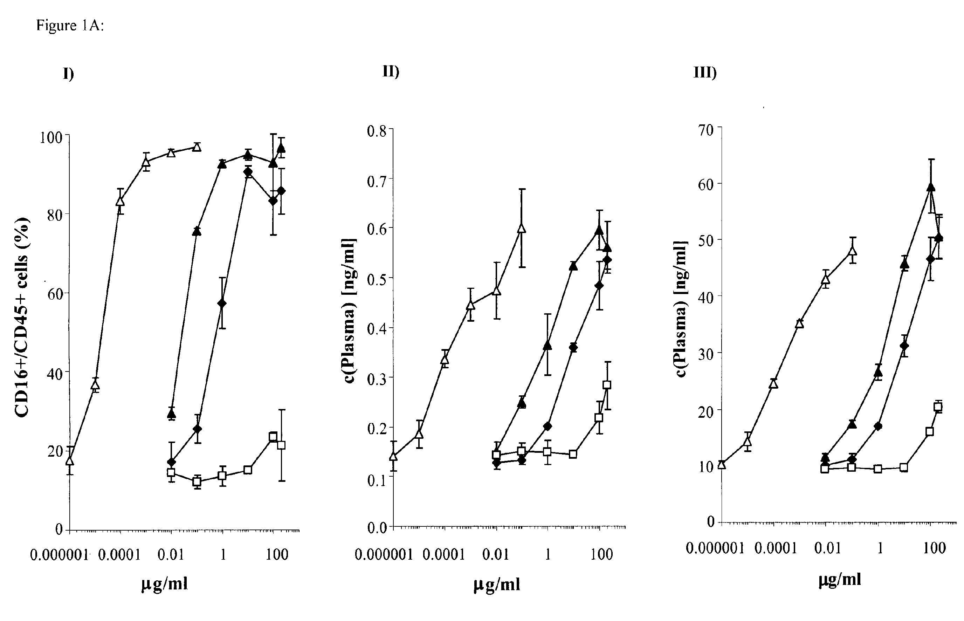

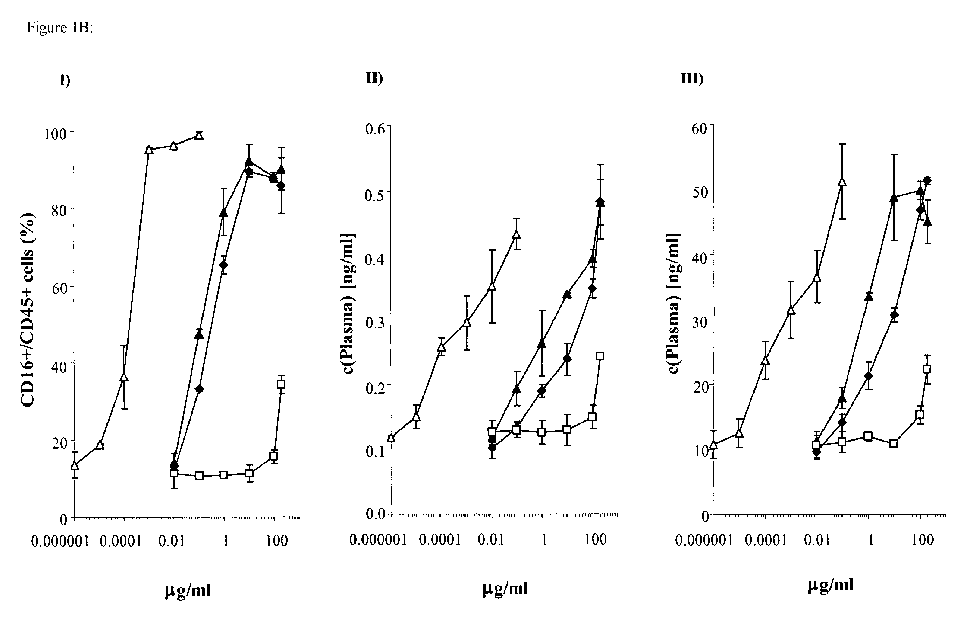

Figure 1 shows a graphical representation of the activation of CD 11 b+ neutrophils (I) and the measured release of PTX-3 (II and III) in the whole blood after exposure to MabCampath®, Synagis®, Orthoclone OKT®3 or LPS . The whole blood samples were collected from two different individuals Donor 1 (Figure 1A) and Donor 2 (Figure 1B) and were each treated for 2 hrs with (0.001 - 100 ng/ml) LPS (Δ), or the positive control mAb MabCampath^{®} (◆), the negative control Ab Synagis^{®} (□) and the comparator mAb Orthoclone OKT3^{®} (▲) in the concentration range of 0.01 - 200 µg/ml corresponding to drug exposure levels *in vivo* typically attained during first-in-man infusions of therapeutic proteins. CD11b expression was determined by FACS (I) and PTX3 release was measured by colorimetric (II) or electrochemiluminescent (III) custom sandwich ELISA in the plasma (obtained by removal of blood cells from the whole blood sample).

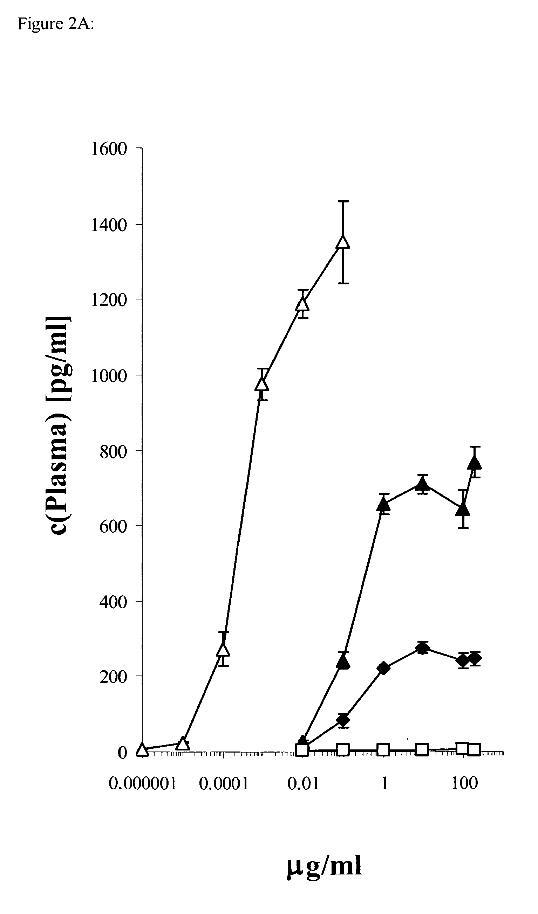

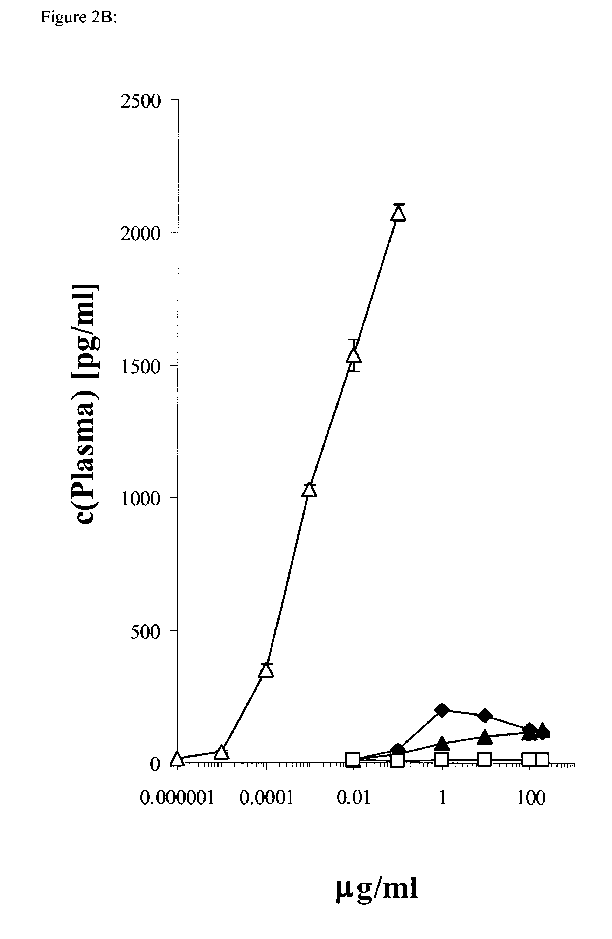

Figure 2 shows a graphical representation of the release of TNF-α in the whole blood after exposure to MabCampath®, Synagis®, Orthoclone OKT®3 or LPS. The whole blood samples were collected from two different individuals Donor 1 (Figure 2A) and Donor 2 (Figure 2B) and were each treated for 2 hrs with the positive control mAb MabCampath® (◆), the negative control Ab Synagis® (□) and the comparator mAb Orthoclone OKT®3 (▲), or with (0.001 - 100 ng/ml) LPS (Δ). CD11b and PTX3 concentrations were measured in the plasma (obtained by removal of blood cells from the whole blood sample). Typically, mAb MabCampath® triggered robust TNF-α release in both donors, attaining peak levels at 1 µg/ml, whereas mAb Synagis® was negative at any concentration in both donors. MAb Orthoclone OKT®3 displayed dose-dependent increase in TNF-α only in blood of one of the donors (Figure 2A), but not in the other donor (Figure 2B).

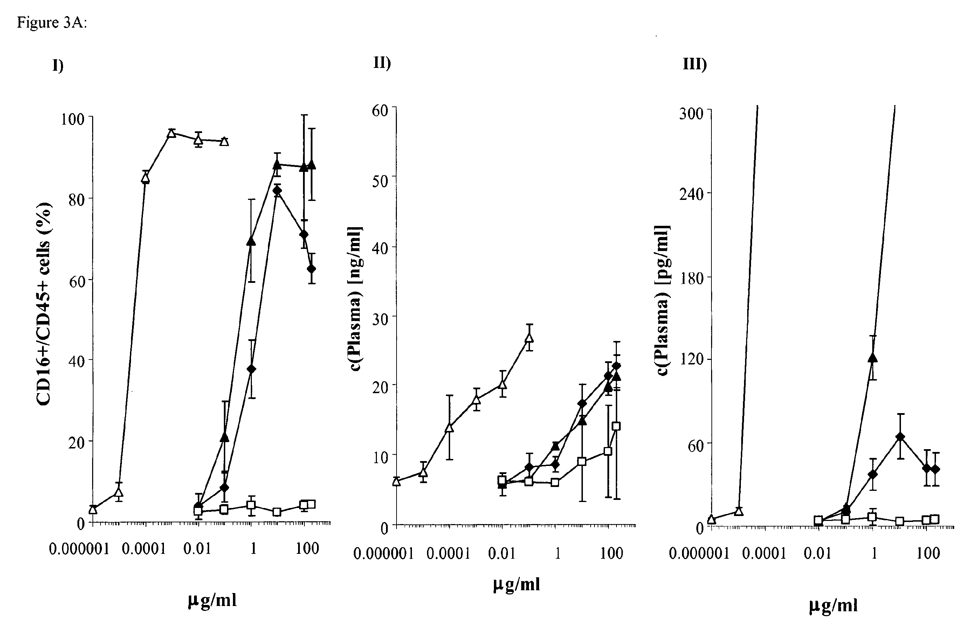

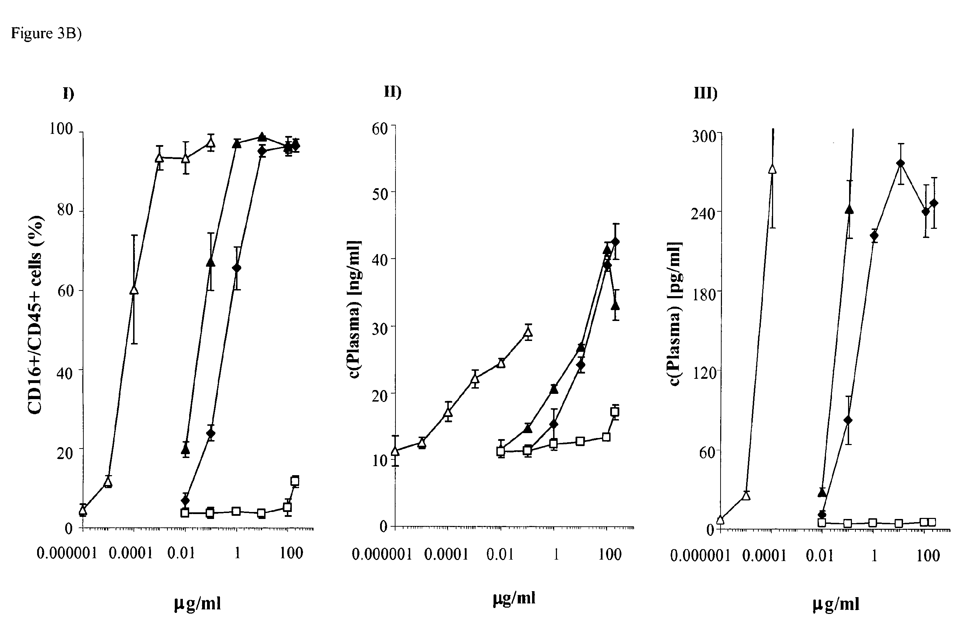

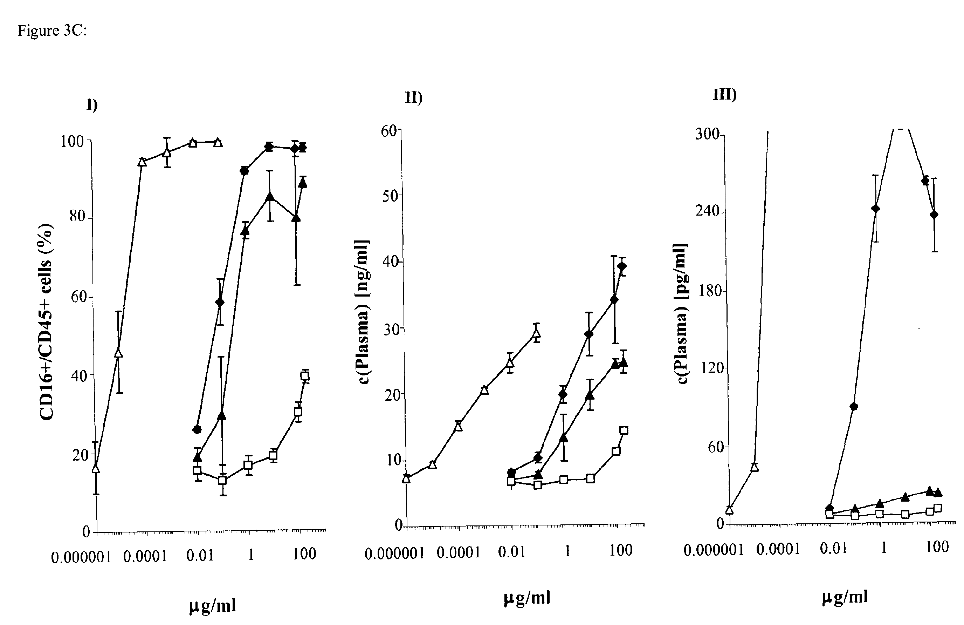

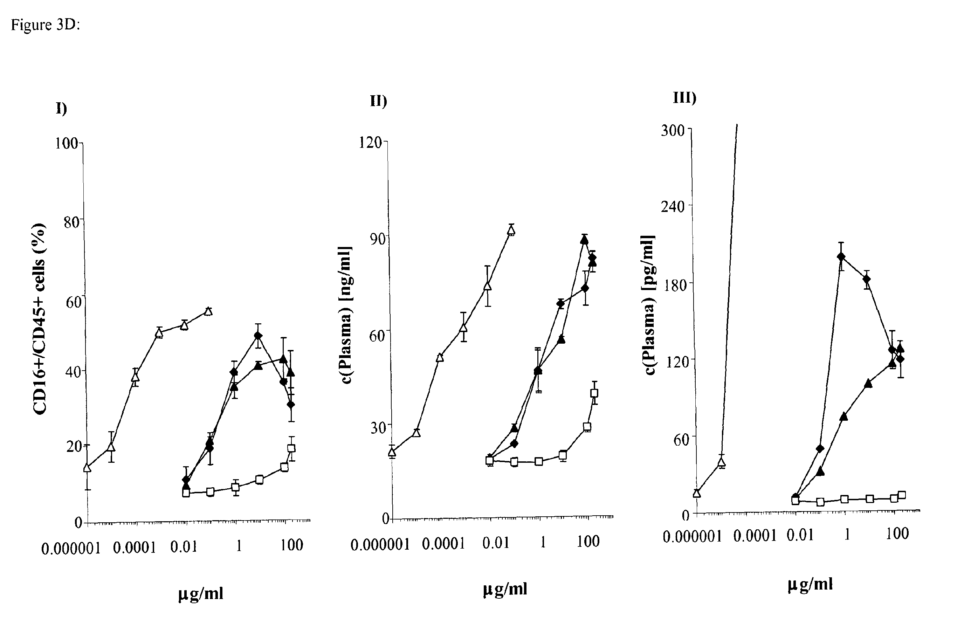

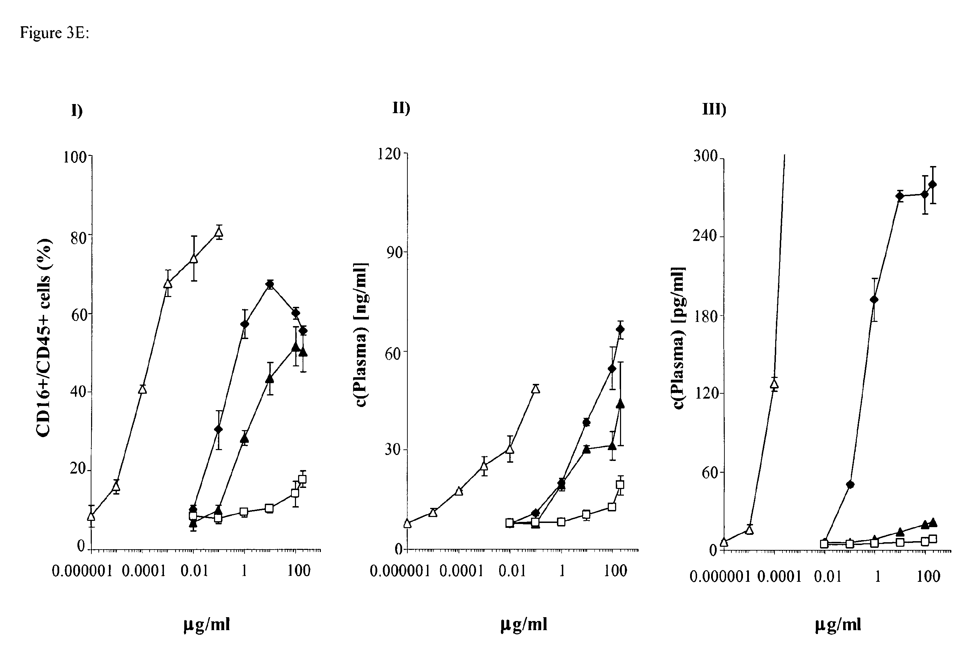

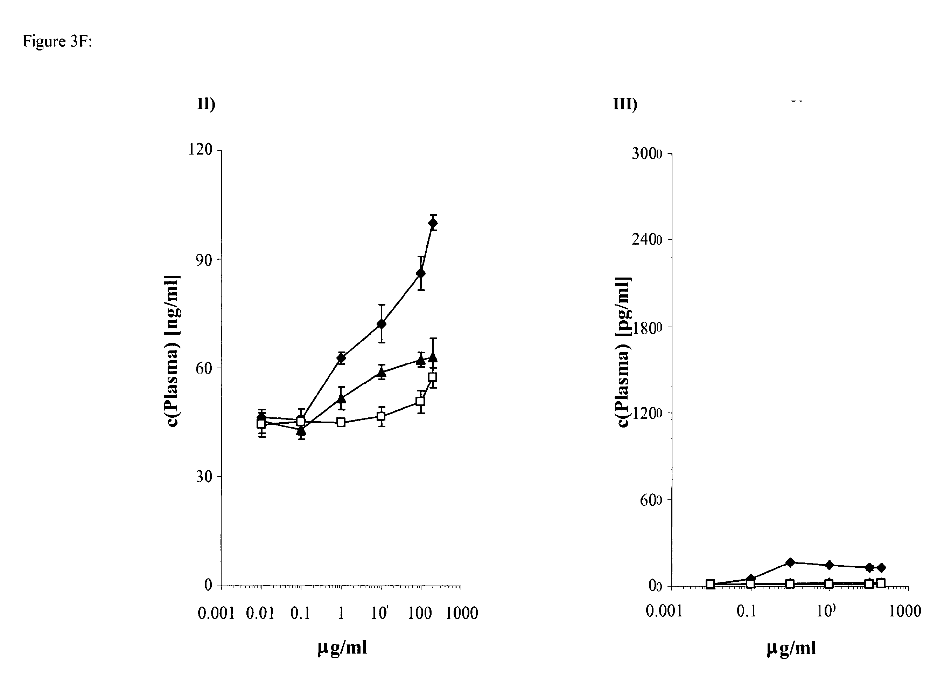

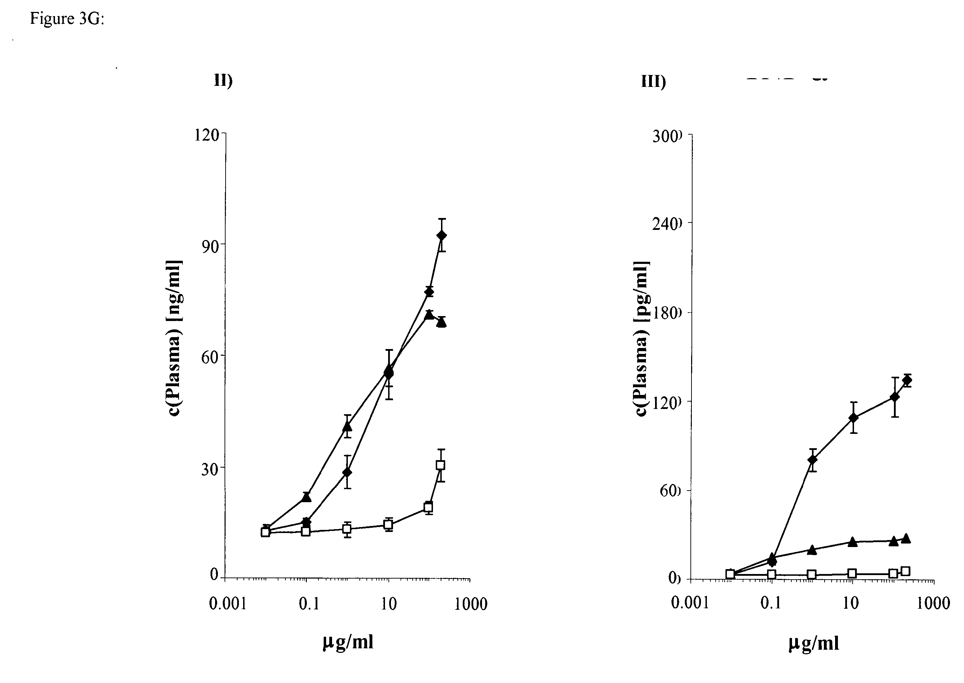

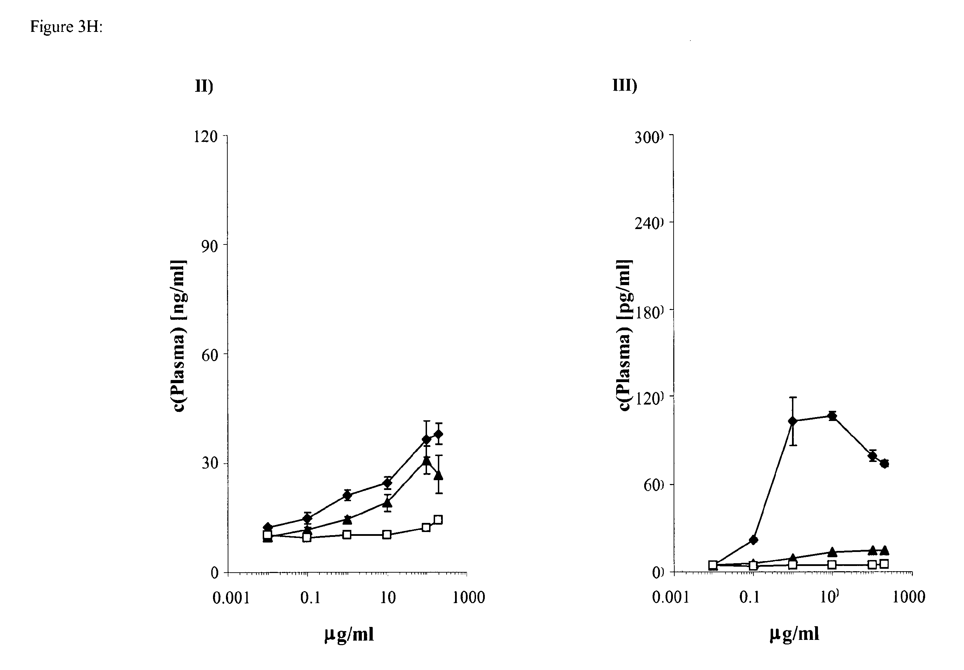

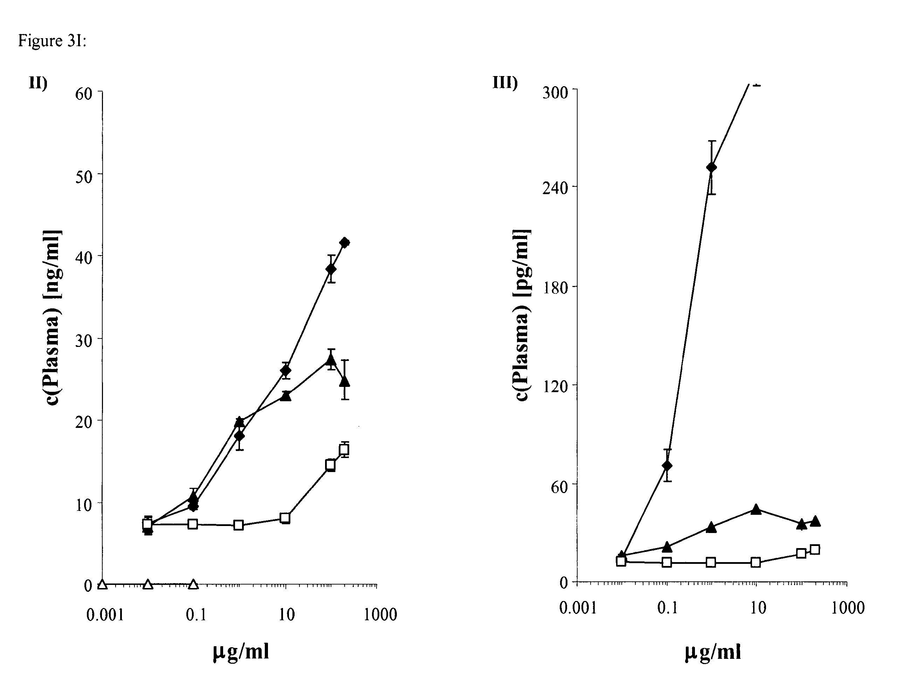

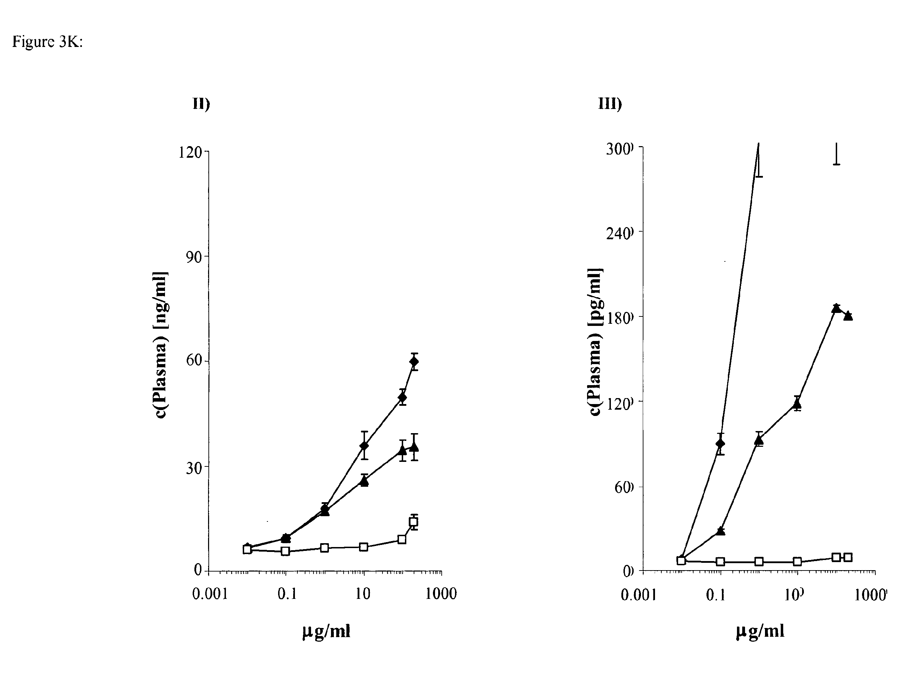

Figure 3 shows a graphical representation of the measured activation of CD11b+ neutrophils (I) and the measured release of PTX3 (II) and TNF-α (III) in whole blood samples from 5-10 healthy individual which were analysed after 2 hours in the presence of different concentrations (200µg/ml, 100µg/ml, 1µg/ml, 1µg/ml, 0.1 µg/ml, 0.01 1µg/ml) of mAb MabCampath® (◆), Orthoclone OKT®3 (▲), Synagis® (□) and LPS (Δ). PTX3 and TNF-α were measured in the plasma (obtained by removal of blood cells from the whole blood sample). A): Donor 1, B): Donor 2, C): Donor 3, D): Donor 4, E): Donor 5, F): Donor 6, G): Donor 7, H): Donor 8, I): Donor 9, K): Donor 10

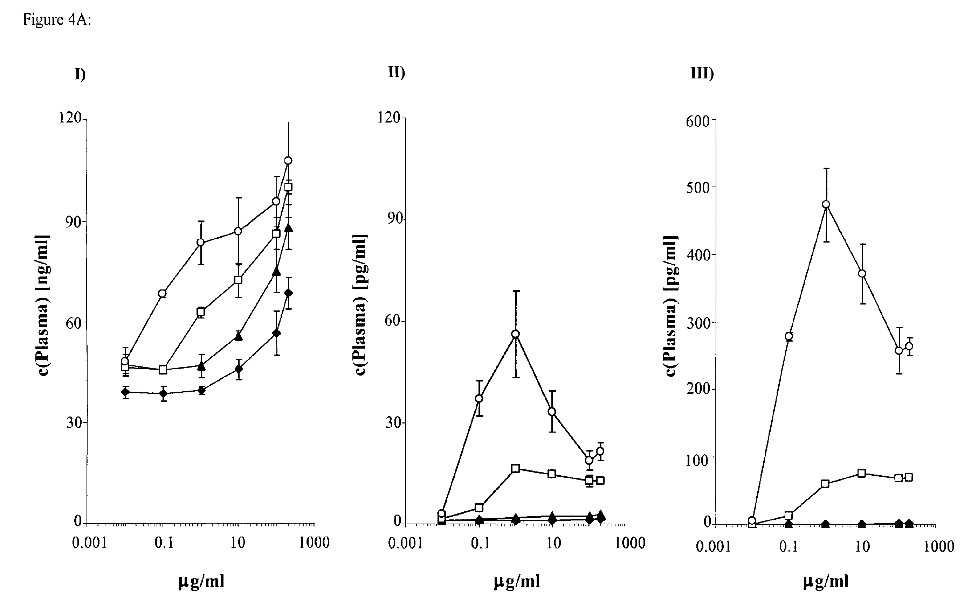

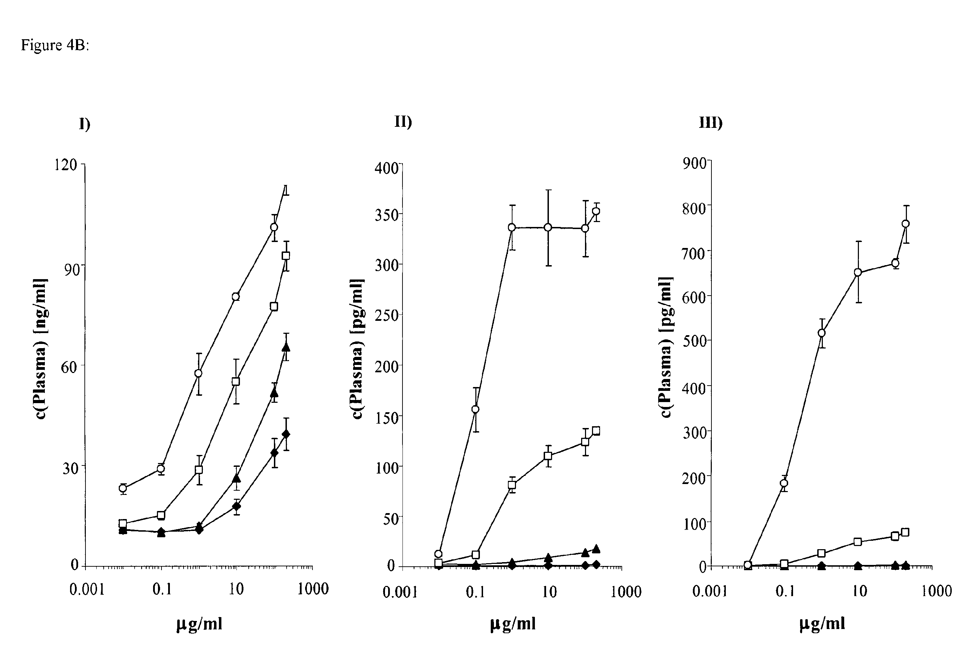

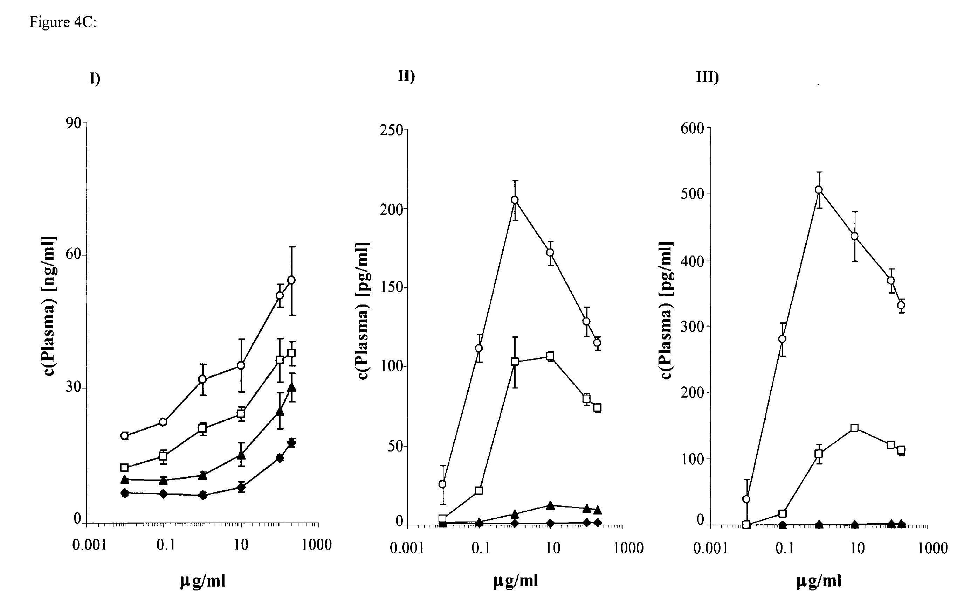

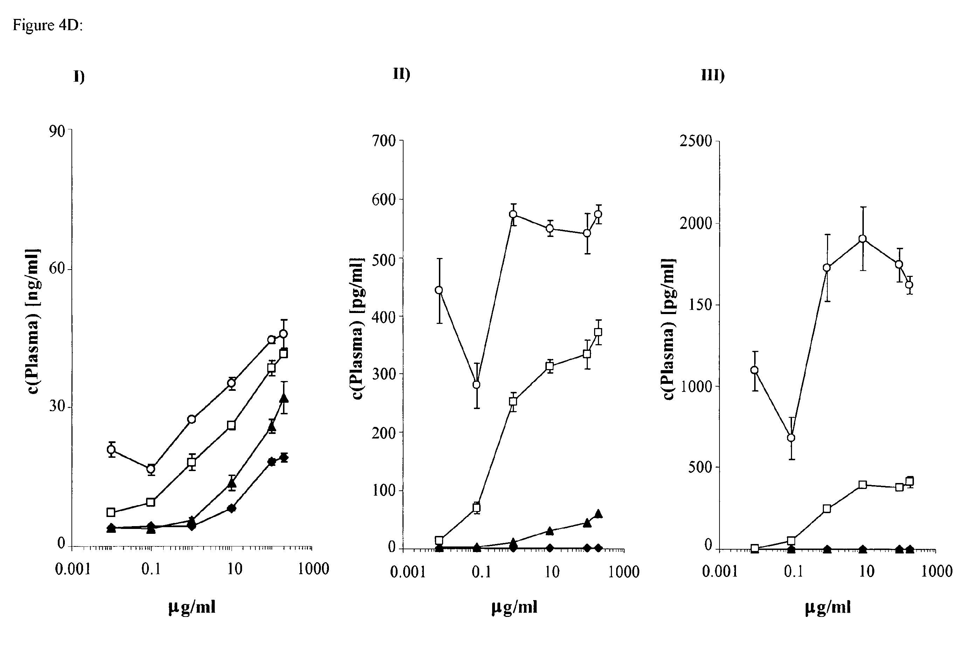

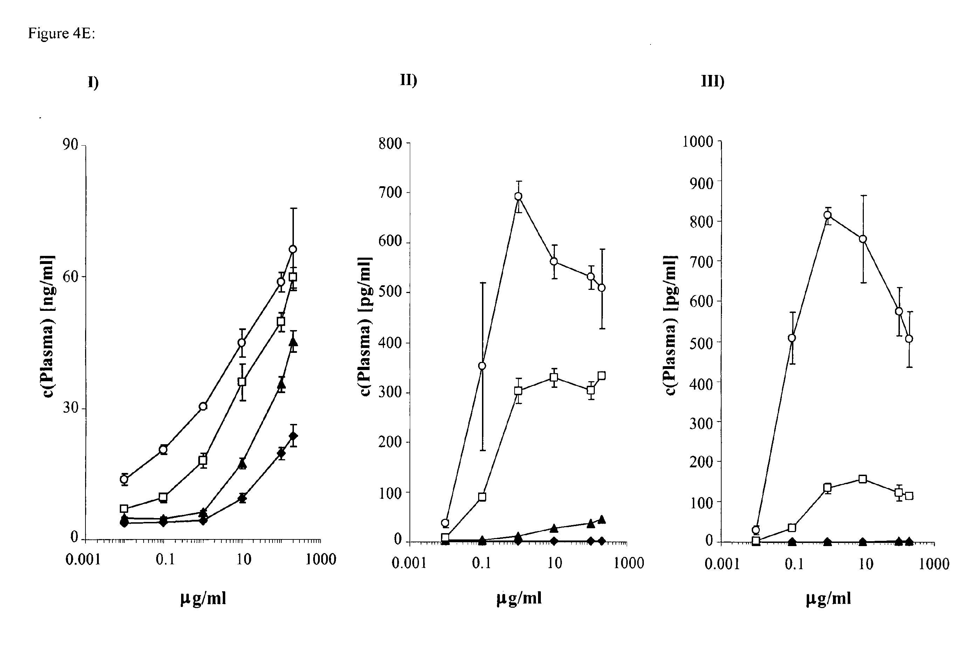

Figure 4 shows a graphical representation of the kinetics of the release of PTX3 (I), TNF-α (II) and IFN-γ (III) after exposure to MabCampath®. Y-axis: measured concentration of PTX3, TNF-α and IFN-γ in the plasma; X-axis: concentrations MabCampath® added to the whole blood sample (200µg/ml, 100µg/ml, 10µg/ml, 1 µg/ml, 0.1 µg/ml, 0.01 µg/ml). Human whole blood samples were incubated with MabCampath® for 30 min (◆), 1 hr (▲), 2 hr (□) and 4 hr (○). Results for 5 healthy donors are shown: A): Donor 6, B) Donor 7, C) Donor 8, D) Donor 9, E) Donor 10.

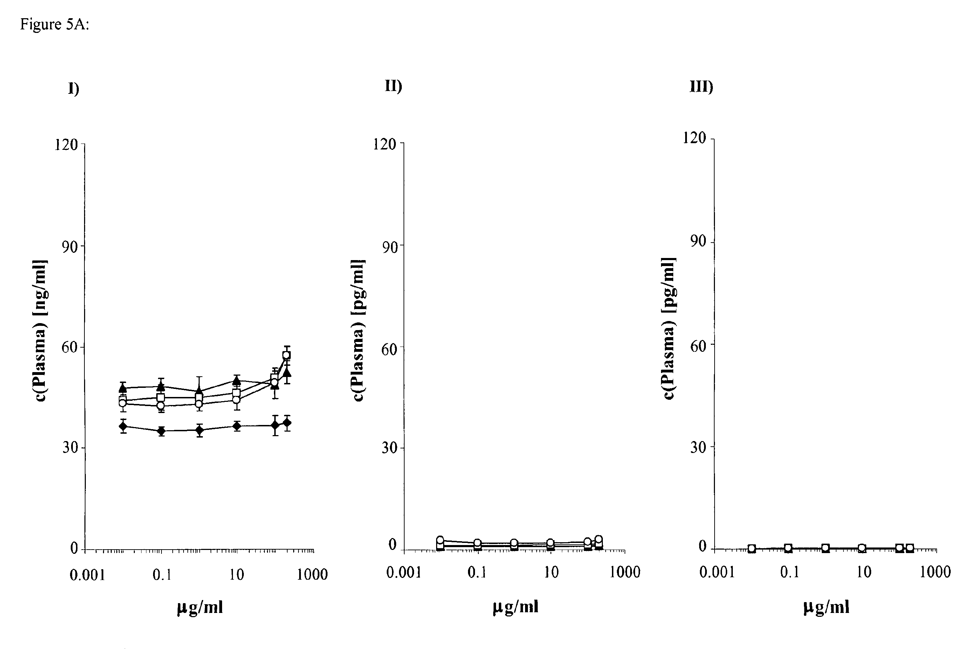

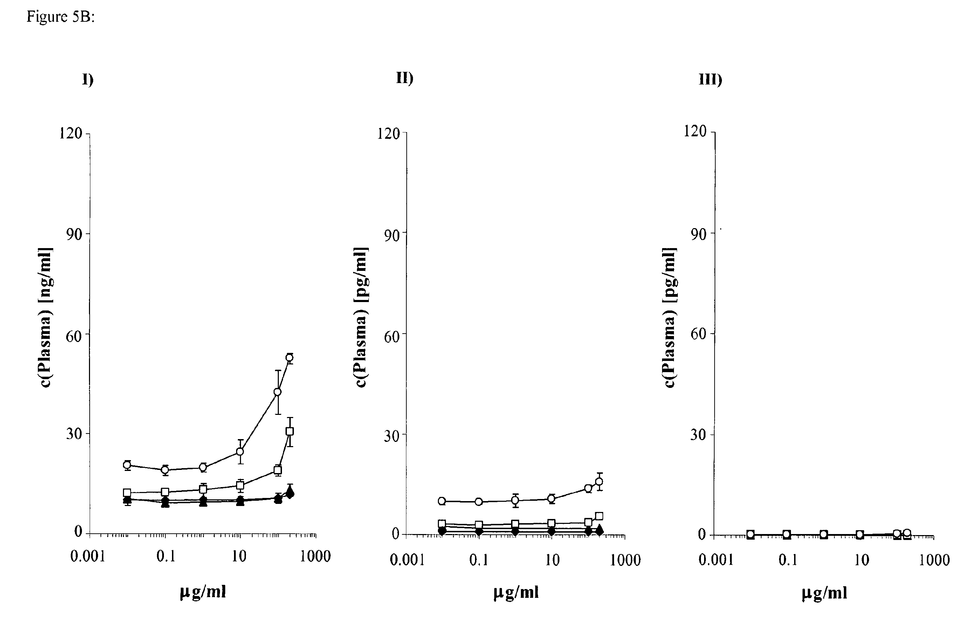

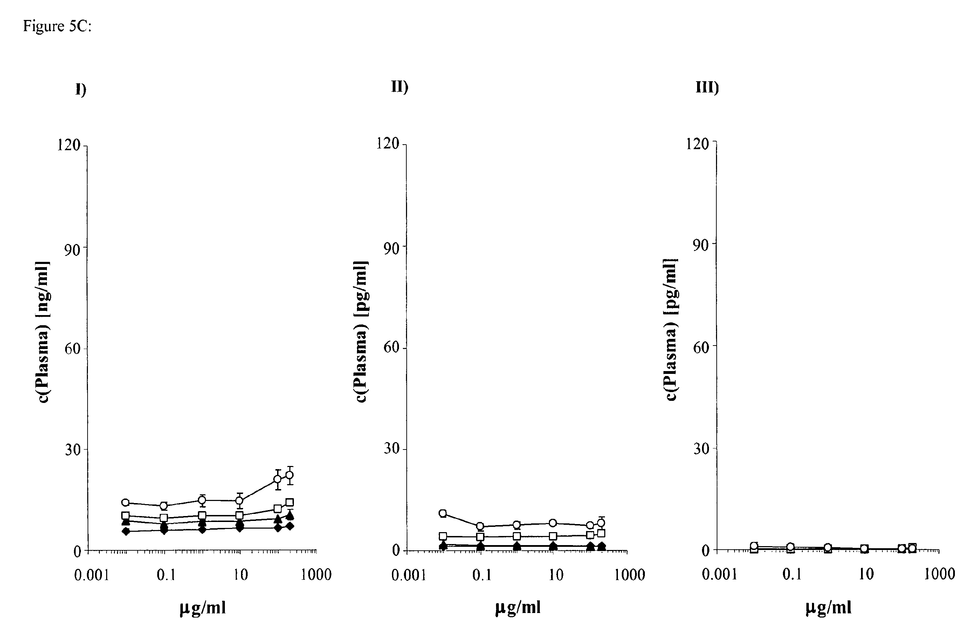

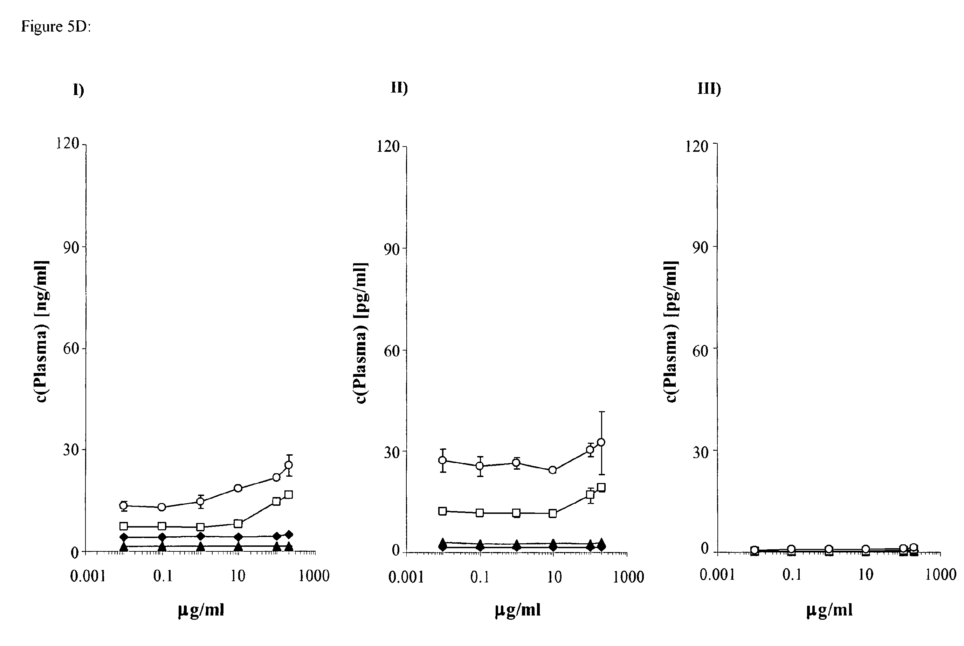

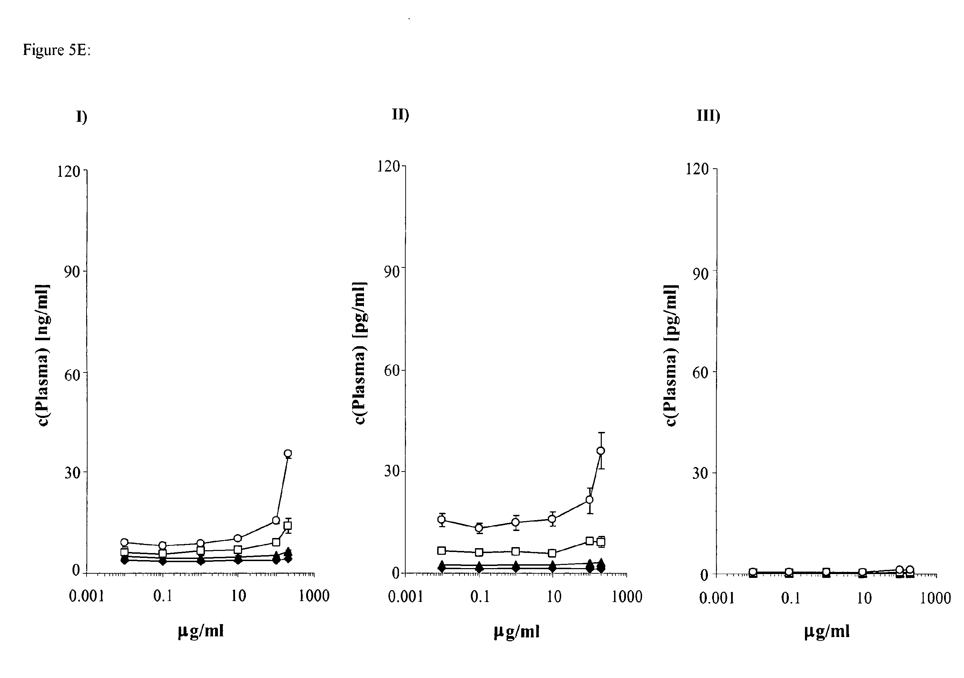

Figure 5 shows a graphical representation of the kinetics of the release of PTX3 (I), TNF-α (II) and IFN-γ (III) after exposure to Synagis®. Y-axis: measured concentration of PTX3, TNF-α and IFN-γ in the plasma; X-axis: concentrations of Synagis® added to whole blood sample (200µg/ml, 100ug/ml, 10µg/ml, 1µg/ml, 0.1µg/ml, 0.01µg/ml). Human whole blood samples were incubated with Synagis® for 30 min (◆), 1 hr (▲), 2 hr (□) and 4 hr (○). Results for 5 healthy donors are shown: A): Donor 6, B) Donor 7, C) Donor 8, D) Donor 9, E) Donor 10.

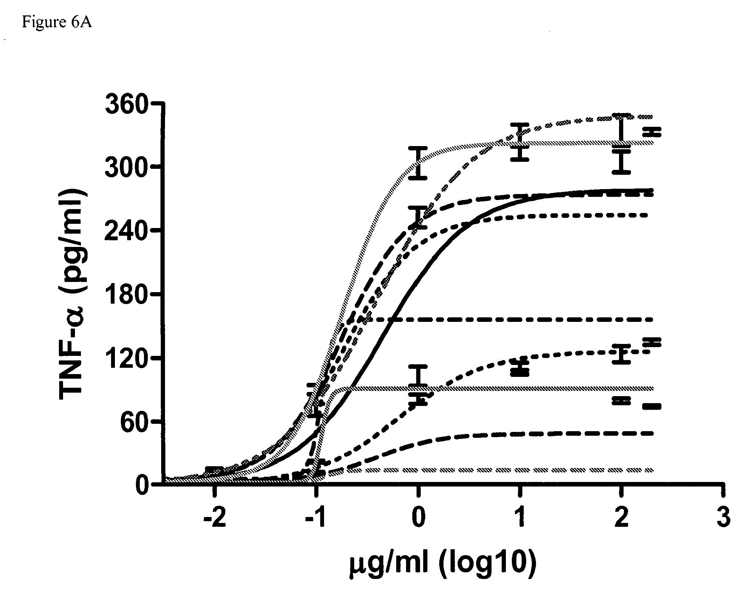

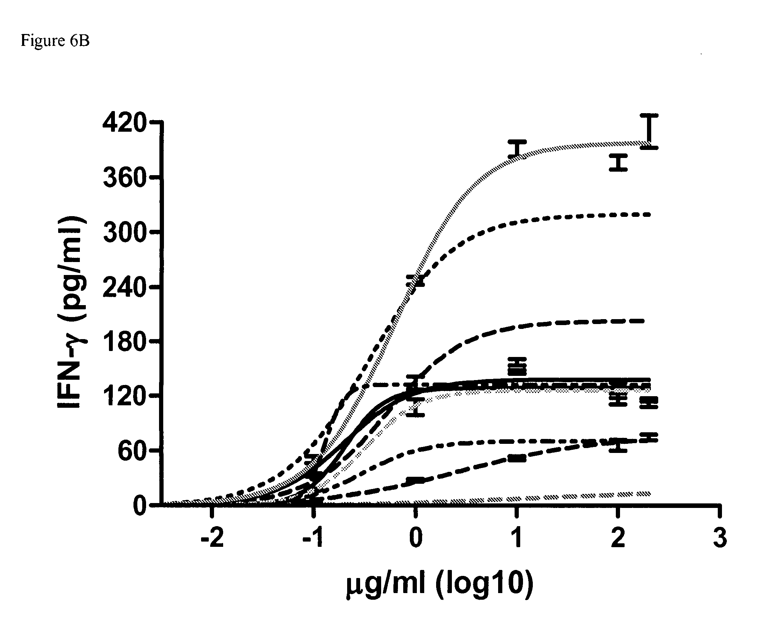

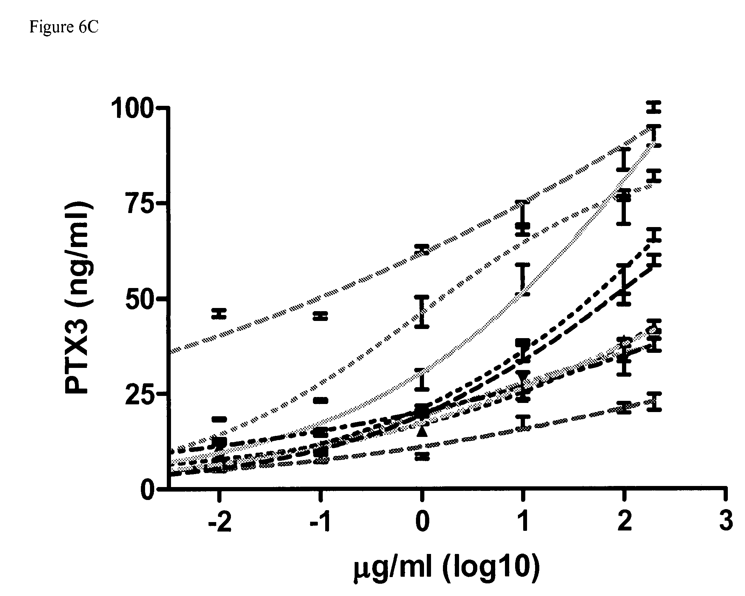

Figure 6 shows graphical representations of the dynamic range of TNF-α, (Figure 6A), IFN-γ (Figure 6B) and PTX3 release (Figure 6C). For the determination of the dynamic range, blood samples from 10 healthy donors were incubated for 2 hours in the presence of the indicated concentrations of MabCampath®. Each curve represents the data collected from one of the donors 1-10.

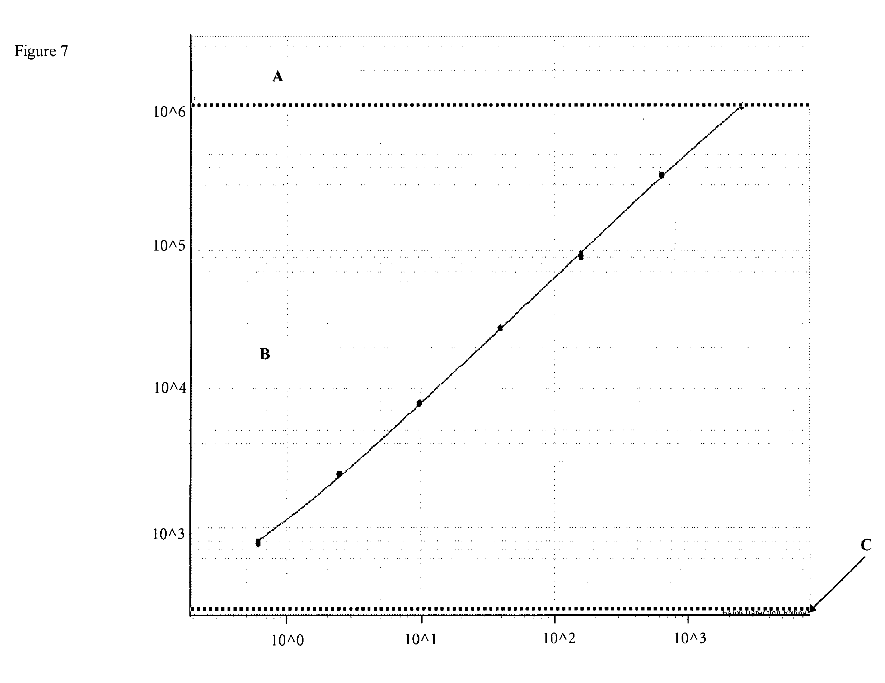

Figure 7 shows a graphical representation of the detectable range of PTX with ECL ELISA. Measurements were made in the plasma obtained by removing the blood cells from the whole blood samples collected from donors 1 to 10. The standard plot shows the linearity of the measured standards over the concentration frame and detection range. A: indicates Signal Above detection range, B: indicates Signal In detection range; C: indicates Signal Below detection range. X-axis: concentrations (pg/mgl), Y-axis: Signal intensity

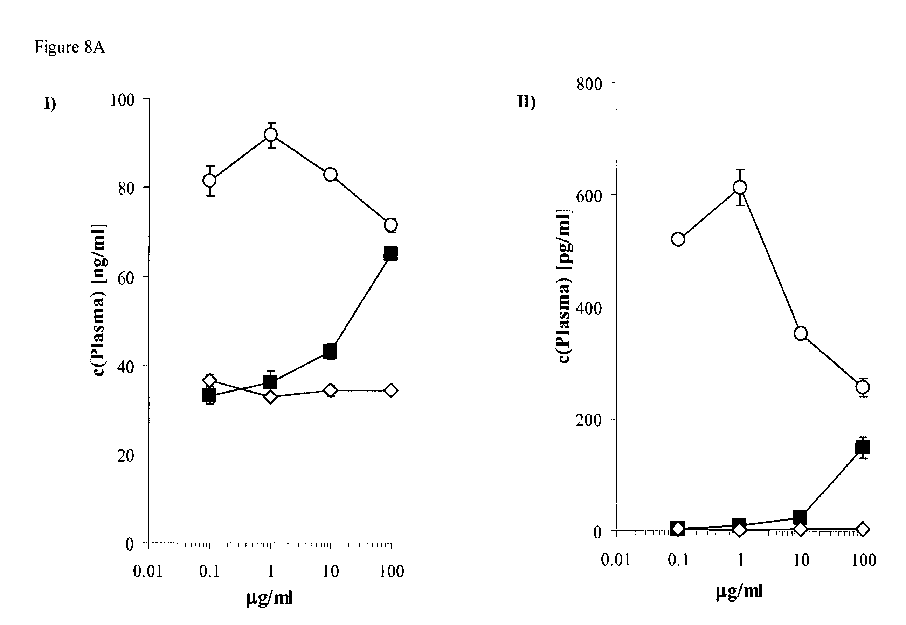

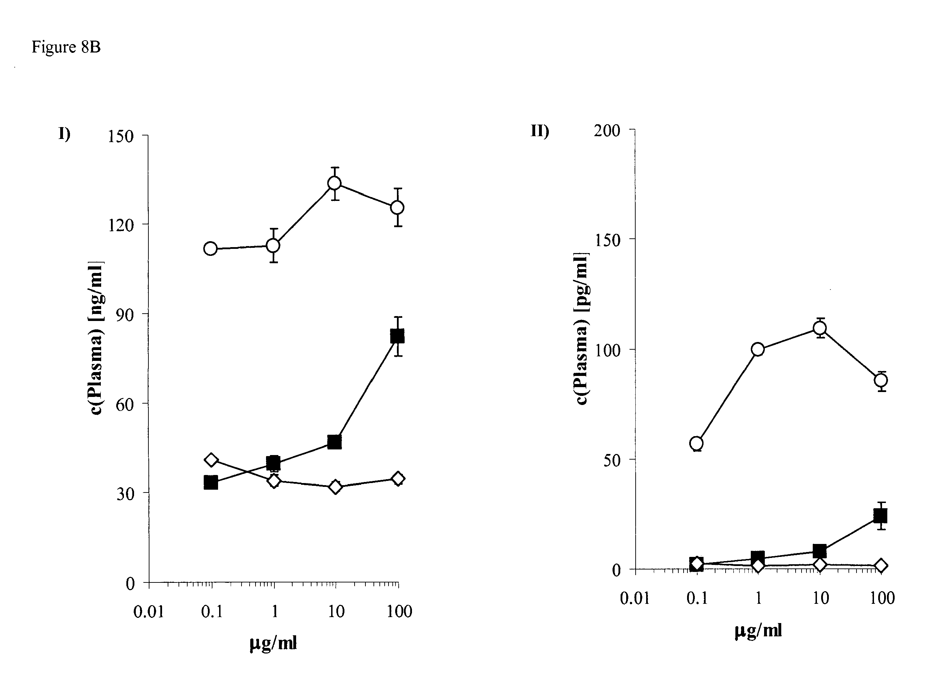

Figure 8 shows a graphical representation of the release of PTX3 after exposure to a TGN11412-like antibody, MabCampath (positive control) and Eribitux (negative control). Blood from 2 different individuals was treated for 24 hrs with the positive control mAb MabCampath^{®} (○), or the negative control Ab Erbitux^{®} (◊) and the comparator TGN1412-like mAb (■) in the concentration 0.1, 1.0, 10 and 100 µg/ml. PTX3 (I) and TNF-α release (II) were measured by electrochemiluminescent custom sandwich ELISA. Figure 8A: Donor A; Figure 8B: Donor B.

### Examples:

Commercially available reagents referred to in the examples were used according to manufacturer's instructions unless otherwise indicated.

### Example 1:

### A) Materials and Methods

### Test substances

As the anti-CD28 mAb TGN1412 that give rise to most severe CRS (Suntharalingam G, et al., Cytokine storm in a phase 1 trial of the anti-CD28 monoclonal antibody TGN 1412. N Engl J Med. 355 (2006): 1018-28), was not publicly available, we utilized utilized a remanufactured anti-CD28 human IgG, TGN1412-like antibody, the anti-CD3 mAb Muromonab-CD3 (Orthoclone OKT®3) (Chatenoud L, Ferran C, Legendre C. In vivo cell activation following OKT3 administration. Systemic cytokine release and modulation by steroids. Transplantation. 49 (1990): 697-702) and, in particular, the anti-CD52 mAb Alemtuzumab (MabCampath®) as reference standards (Wing MG, et al. Mechanism of first-dose cytokine-release syndrome by CAMPATH® 1-H: involvement of CD 16 (FcgammaRIII) and CD11a/CD18 (LFA-1) on NK cells. J Clin. Invest. 98 (1996): 2819-26), in accordance to the Expert Scientific Group on Phase One Clinical Trials (ESG Final Report:EXPERT SCIENTIFIC GROUP ON PHASE ONE CLINICAL TRIALS, Final Report, Department of Health (UK), 30. November 2006).

**Comparator: Muromonab-CD3** (Orthoclone OKT®3), anti-CD3 mouse IgG2a, Jansen Cilag AG, Switzerland, Lot. no. BN 6GSTV00, Expiry date: April 2007, stock 10 mg/ml (concentrated from 1mg/ml infusion solution with Centricon Ultracel YM-100. Cat. No. 4211, Micon), concentration and purity was checked by Bradford analysis and SDS-PAGE. Stored in aliquots at 4°C, further diluted in PBS (pH 7.4) (cf. "whole blood assay").

The murine IgG2a anti-CD3 mAb Orthoclone OKT®3 which gives rise to CRS in renal transplant patients is utilized as an additional reference drug. It was positive for TNF-α release in 40% of the cases tested (Figures 2, 3 and 6).

Remanufactured anti-CD28 human IgG, TGN1412-like antibody, (based on TeGenero **TGN1412** published sequence in US2006/0286104 A1) stock 11.2 mg/ml , concentration and purity were checked by Bradford analysis and SDS-PAGE. Stored at -80°C, further diluted in PBS.

**Positive control: Alemtuzumab** (MabCampath®), anti-CD52 humanized IgG1, Schering, Switzerland, Lot. no. 42018R, Expiry date: May 2007 and 42019T, Expiry date: December 2007, stock 10 mg/ml, sterile liquid, stored in aliquots at 4°C; further diluted in PBS (cf "whole blood cell assay").

The humanized IgG1 anti-CD52 mAb MabCampath® which is known to trigger first-dose CRS in leukemia, lymphoma and multiple sclerosis patients (Wing MG, et al., Mechanism of first-dose cytokine-release syndrome by CAMPATH® 1-H: involvement of CD16 (FcgammaRIII) and CD11a/CD18 (LFA-1) on NK cells. J Clin. Invest. 98 (1996): 2819-26), is used as a positive control for cytokine release and concomitant activation of neutrophils. MabCampath® induced TNF-α release over the threshold of 60 ± 10 pg/ml (cf "Thresholds") in all but one sample that have been tested (Figures 2, 3 and 6, Table 5). The first donor of the kinetic study reached this threshold of TNF-α secretion only after 4 hours of incubation with MabCampath®.

**Negative Control Antibody: Palivizumab** (Synagis®), anti-RSV humanized IgG1, Abbott Laboratories, Lot. no. 30282 TF, Expiry date: June 2008, stock 100 mg/ml, reconstitution in endotoxin-free water provided with the medicinal product, stored in aliquots at -80°C, further diluted in PBS (cf. "whole blood cell assay").

The humanized IgG1 anti-Respiratory Syncytial Virus (RSV) mAb Synagis®, indicated for congenital heart disease in children, which is not associated to any type of CRS, is used as a negative control. MAb Synagis® was negative for cytokine release in all cases tested (Figures 2, 3 and 6).

Erbitux (Cetuximab®), recombinant human/mouse chimeric monoclonal antibody that binds specifically to the extracellular domain of the human epidermal growth factor receptor (EGFR), for which severe infusion reactions occurred in approximately 3% of patients, stock 2 mg/ml, stored in aliquots at 4°C, further diluted in PBS.

### Drug concentration range

The medicinal products to be analyzed are tested in the concentration range 0.01 - 200 µg/ml (0.01 µg/ml, 0.1 µg/ml, 1 µg/ml, 10 µg/ml, 100 µg/ml and 200 µg/ml, or 0.1 µg/ml, 1 µg/ml, 10 µg/ml, and 100 µg/ml). The range covers in most instances the peak exposure level of the respective product expected for first-in-man dosing, as derived from the respective pharmacokinetic (PK) profile. All reference drugs are analyzed in parallel in the same concentration range, thereby also covering typical plasma exposures of the respective drugs in treated patients. PBS controls were included to determine baseline levels.

Drug concentrations >200 µg/ml are hardly amenable to the WBA due to cytotoxicity of the reference drug, mAb MabCampath®, leading to death of blood borne cells, and due to limitations in the volume that can be added to whole blood by avoiding unacceptable dilution.

### Whole blood cell assay (WBA)

Unless specified, whole blood from healthy blood donors was collected in vacutainer tubes containing lithium heparin as anticoagulant (Blood Donation Center, Swiss Red Cross, Basel, Switzerland, or Roche Pool). Blood was processed within 1-3 hours after collection and co-incubated at 37°C for 30 min up to 4 hours and for 24 hours with increasing concentrations of the relevant medicinal product. Pre-validation experiments revealed optimal performance conditions with blood processing started within 4 hours as otherwise lysis of erythrocytes would disturb the assay. The medicinal products including the reference antibodies MabCampath®, Orthoclone OKT®3 and Synagis® were analysed at the following concentrations: 0.01 µg/ml, 0.1 µg/ml, 1 µg/ml, 10 µg/ml, 100 µg/ml and 200 µg/ml. The TGN1412-like antibody was analysed in the following concentrations: 0.1 µg/ml, 1 µg/ml, 10 µg/ml, 100 µg/ml. 195 µl of blood was added in triplicates to U-bottom wells of 96-well plates containing 5 µl of the items to be tested. These conditions ensure optimal accomplishment in respect to practicability and efficiency to gain at least 70 µl of plasma and sufficient cells to conduct PTX3, multi-cytokine measurement in plasma and eventually CD11b flow-cytometric cells analysis, respectively. Endogenous activation of blood cells was assessed by including controls containing PBS (Phosphate-Buffered Saline, pH 7.4).

After 30 min to 4 hours/24 hours incubation at 37°C in a incubator with 5% CO₂, plates were centrifuged at 1800g for 5 min. Supernatant (containing approximately 70 µl plasma) was carefully removed and frozen in aliquots at -70°C. 70 µl PBS (pH 7.4) was added to the cell pellet and mixed gently.

### TNF-α and IFN-γ, Multiplex assay (MSD)

For determination of cytokines in plasma, the plasma was thawed. Pre-tests revealed that levels of cytokine did not differ between fresh and thawed plasma samples. Cytokine concentration was determined by an ultra-sensitive human cytokine assay according to the manufacturer's protocol (MesoScale Discovery, Gaithersburg MA, USA) (Human Cytokine Assay, MS2400 custom plate human TNF-α and IFN-γ 2erPlex from MSD). Plates were analyzed on a Sector Imager 2400 using MSD Discovery workbench software 2.0 (MesoScale Discovery, Gaithersburg MA, USA).

Data are presented as the mean of measurements on plasma supernatants from triplicate wells. Results are expressed as pg/ml.

### Fluorescence-activated cell sorter (FACS)

For FACS-analysis 50 µl of whole blood cells (see whole blood cell assay) were incubated for 30 min at room temperature with the antibodies CD45 PerCP, (Cat. No. 345809, BD Pharmingen), CD11b PE (Cat. No. 555388, BD Pharmingen) and CD16 FITC, (Cat. No. 554406, BD Pharmingen). After incubation time 450 µl of FACS Lysis Solution (Cat. No. 349202, BD Pharmingen) was added to the cell suspension. Stained and fixed cells were stored at room temperature in the dark and analysed by flow cytometry (BD FACS Calibur; CellQuestPro 4.0.2.Software, BD Biosciences San Jose CA, USA) the next day for upregulation of CD11b on CD16+/CD45+ neutrophils (Nicholson et al. A novel flow cytometric assay of human whole blood neutrophil and monocyte CD11b levels: upregulation by chemokines is related to receptor expression, comparison with neutrophil shape change, and effects of a chemokine receptor (CXCR2) antagonist.Pulm Pharmacol Ther. 20 (2007): 52-9.).

### Colorimetric ELISA for PTX3 in human plasma

A sandwich ELISA was developed using PTX₃ human detection set (Cat. No. ALX-850-299, Alexis Biochemicals (ALEXIS Corporation, Lausen, Schweiz). After overnight coating at 4°C with capture antibody (700 ng/ml), 96-well plates (Cat. No. 446612, Nunc Maxisorp^{™}, Nunc A/S) were washed with PBS-0.05% tween and blocked with PBS-0.2% gelatin buffer for 1-2 hr at 37°C. Calibrators were prepared by diluting recombinant human PTX3 protein in PBS-tween-2% BSA (Tween 0.05%, PBS pH7.4). Samples and calibrators were incubated 2 hr at 37°C followed by biotinylated detection antibody (50 ng/ml) 1 hr at 37°C. Colorimetric reaction was initiated incubating the samples with streptavidin-peroxydase (Cat. No. 7100-05, Southern Biotech Birmingham, USA) for 45 min followed by TMB (Tetramethyl benzidine, Cat. No. T4444, Sigma Saint Louis, USA) for 15 min, then stopped with concentrated sulphuric acid. Plates were read at 450nm on an ELISA reader using SoftMax Pro software (Molecular Devices, USA).

### ECL ELISA for PTX3 in human plasma

MSD QuickPlex custom plates (MesoScale Discovery, Gaithersburg MA, USA) and immunoassay were designed for PTX3 measurement by electrochemiluminescence, using PTX; human detection set (Cat. No. ALX-850-299, Alexis). The capture antibody (1 µg/ml) was conjugated to a QuickPlex linker (Cat. No. K15A06-2, MesoScaleDiscovery) and the immunoassay was conducted according to MSD instructions (MesoScale Discovery, Gaithersburg MA, USA). Plates were analyzed on a Sector Imager 2400 Reader (MesoScale Discovery, Gaithersburg MA, USA).

Data are presented as the mean of measurements on plasma supernatants from triplicate wells. Results are expressed as ng/ml.

### Definition of the threshold (positive control)

Samples were considered "positive" (= elevated risk of occurrence of CRS) with respect to the induction of cytokine by the respective medicinal product, provided that two criteria were fulfilled:
(i) A TNF-α level was measured that was above the cut-point of 60 ± 10 pg/ml. This threshold is derived from *in vivo* data collected from plasma of multiple sclerosis patients that developed CRS after first infusion of MabCampath® (Moreau T, et al. Transient increase in symptoms associated with cytokine release in patients with multiple sclerosis. Brain. 119 (1996): 225-37.). 60 ± 10 pg/ml was the lowest TNF-α level reported in the respective patient cohort to be associated to severe CRS. TNF-α values in a similar range were found in the context of oncology patients displaying CRS after first infusion of Rituximab (Winkler U, et al., Cytokine-release syndrome in patients with B-cell chronic lymphocytic leukemia and high lymphocyte counts after treatment with an anti-CD20 monoclonal antibody (Rituximab, IDEC-C2B8). Blood. 94 (1999): 2217-24).
(ii) The percentage of CD 11b+ neutrophils exceeded 30%.

This threshold is explained by the fact that even in the absence of cytokine release, activation of neutrophils is observed at a certain extent, occasionally attaining values up to 25 - 30%, depending on the donor.

### Statistical evaluation

Mean values and standard deviations were calculated from triplicates. The evaluation of the dynamic range and the calculation of the coefficient of variance were performed by GraphPad prism 4 (GraphPad Software Inc, La Jolla, USA.). The LLOD and LLOQ were calculated with LOD Fit 1.0 (Roche Pharma Informatics).

### Relation of in vitro assessment to cytokine release in vivo

The WBA protocol foresees for OKT® co-incubation at 37°C for up to 4 hours of heparinated blood from healthy individuals or patients, within 1 - 3 hrs after collection, with increasing concentrations of the relevant medicinal product. The plasma derived from those samples is subsequently analysed for the release of PTX3 and of the pro-inflammatory cytokines TNF-α and IFN-γ.

This protocol mimics as closely as possible the situation *in vivo:* antibodies that have been reported to trigger CRS, induce systemic release of TNF-α and/or IFN-γ and potentially other cytokines and chemokines, such as IL-6, IL-8, within 0.5 - 4 hrs after the onset of the first infusion (Moreau T, et al., Transient increase in symptoms associated with cytokine release in patients with multiple sclerosis. Brain. 119 (1996): 225-37; Suntharalingam G, et al., Cytokine storm in a phase 1 trial of the anti-CD28 monoclonal antibody TGN1412. N Engl J Med. 355 (2006): 1018-28). Additionally in patients with systemic inflammatory response syndrome or undergoing a septic shock, PTX3 plasma levels dramatically increase during the first 24 hours and correlate with clinical score and disease severity (Muller B, et al., Circulating levels of the long pentraxin PTX3 correlate with severity of infection in critically ill patients, Crit Care Med. (2001) 29:1404-1407). Altogether these soluble factors are thought to trigger and reflect a sizeable number of symptoms of variable severity, apparently depending on the amount, locality, timing and type of cytokines being released and circulated via the blood stream.

### Endpoints

Two types of endpoints have been established to address the risk of drug-induced cytokine release: secretion of key pro-inflammatory cytokines, such as TNF- α and IFN-γ and upregulation of the adhesion molecule CD11b on neutrophils:

### a. Pro-inflammatory cytokines TNF-α and IFN-γ

Among other cytokines, the cytokines TNF-α, IL-6 and IFN-γ have repeatedly been described in the context of first-infusion-related CRS. Primarily TNF-α appears to be the kinetically first and pace-making cytokine to be released by the respective drugs, with the amplitude of TNF-α release correlating to a certain extent with the severity of CRS (Suntharalingam G, et al. Cytokine storm in a phase 1 trial of the anti-CD28 monoclonal antibody TGN1412. N Engl J Med. 355 (2006): 1018-28; Winkler U, et al., Cytokine-release syndrome in patients with B-cell chronic lymphocytic leukemia and high lymphocyte counts after treatment with an anti-CD20 monoclonal antibody (Rituximab, IDEC-C2B8). Blood. 94 (1999): 2217-24).

The indicated cytokines are determined by use of the MSD Ultra-sensitive human cytokine assay (Meso Scale Discovery, Gaithersburg, Maryland, USA) that allows detection of multiple analytes in a small volume sample. Compared to conventional ELISA, the MSD system has the advantage of (i) the broad dynamic range and high sensitivity of sulfo-TAG electroluminescent detection (ii) efficient capture of analytes via specific antibodies immobilized in a patterned array on multi-spots plates and (iii) complete traceability back to plate manufacturing records and antibody spot pattern via a unique bar code system.

### b. Adhesion molecule CD11b on neutrophils

Cytokine release is often coupled to capillary leakage and diapedesis of blood cells into peripheral tissue leading to a drop of blood pressure and sometimes to multi-organ failure (Suntharalingam G, et al. Cytokine storm in a phase 1 trial of the anti-CD28 monoclonal antibody TGN1412. N Engl J Med. 355 (2006): 1018-28.). Activated neutrophils which express elevated levels of the adhesion molecule CD11b (Nicholson GC, et al., A novel flow cytometric assay of human whole blood neutrophil and monocyte CD11b levels: upregulation by chemokines is related to receptor expression, comparison with neutrophil shape change, and effects of a chemokine receptor (CXCR2) antagonist. Pulm Pharmacol Ther. 20 (2007): 52-9), appear to belong to the subset of immune cells that are involved in capillary leak and its consequences. Hence, measurement of the fraction of CD11b+ neutrophils links the release of pro-inflammatory cytokines to potential downstream events, such as activation of neutrophils, adherence of neutrophils to capillary walls and concomitant opening of the endothelial lining leading to capillary leak syndrome.

The fraction of activated neutrophils can be determined by multi-color flow-cytometry, gating on the subset of CD45+/CD16+ blood cells. Changes relative to baseline in the percentage of CD11b+ cells upon addition of the respective drug were not re-evaluated.

### Thresholds

### Cytokines:

The amount of secreted TNF-α in plasma apparently correlates with the number and severity of symptoms associated to CRS upon first dosing. Consequently, the amount of TNF-α, as determined in the MSD test, was considered most relevant for risk assessment. According to data obtained *ex vivo* from multiple sclerosis patients treated with MabCampath® (Moreau T, et al., Transient increase in symptoms associated with cytokine release in patients with multiple sclerosis. Brain. 119 (1996): 225-37.) and lymphoma or leukemia patients treated with Rituximab (Winkler U, et al., Cytokine-release syndrome in patients with B-cell chronic lymphocytic leukemia and high lymphocyte counts after treatment with an anti-CD20 monoclonal antibody (Rituximab, IDEC-C2B8). Blood. 94 (1999): 2217-24), a TNF-α level of 60 ± 10 pg/ml was considered as a reasonable cut-point to differentiate positive from negative samples. This threshold value corresponds to the minimal level of TNF-α which was sufficient for the induction of a mild version of CRS.

### CD11b:

In the absence of TNF-α or one of the other cytokines the subset of CD11b+ neutrophils revealed to attain baseline levels of up to about 30% under the conditions of the WBA described here. Consequently, the percentage of 30% was used as the cut-point. Thus, only values >30% of CD11b+ neutrophils were considered a positive signal.

In summary, samples attaining TNF-α levels >60 pg/ml and >30% CD11b+ neutrophils were considered "positive" for the risk of clinically relevant cytokine release.

### Longitudinal stability of cytokine release and neutrophil activation

Drug-induced cytokine release and concomitant neutrophil activation in whole blood from a healthy individual appears to be rather longitudinally stable than meaningful variable. Therefore study of longitudinal stability of cytokine release was not reiterated here.

### Technical qualification

As TNF-α turned out to be in many cases the kinetically first cytokine to be released in patients suffering from CRS, TNF-α appears to be the key cytokine for risk assessment of CRS. Consequently, the technical qualification program, detailed below, primarily refers to data obtained with TNF-α.

An additional cytokine, IFN-γ, has been studied, however, mainly for confirmatory reasons.

The subset of CD11b+ neutrophils has been analysed in parallel in order to evaluate to what extent TNF-α release is accompanied by neutrophil activation, as determined by up- . regulation of the adhesion molecule CD11b. As an innovative approach, PTX3 was measured in human plasma to evaluate its readiness to translate neutrophil activation and to determine whether PTX3 may serve as an adequate parameter for the assessment of the risk of CRS.

MabCampath® was chosen as the main reference drug for the technical qualification of the WBA, as the majority of blood donors displayed moderate to strong cytokine release in the presence of MabCampath®.

### PTX3 measurement and neutrophil activation (results)

### OKT3®

Blood samples from 2 different individuals were treated for 2 hrs with (0.001 - 100 ng/ml) lipopolysaccharide (LPS) or with the positive control mAb MabCampath^{®}, the negative control Ab Synagis^{®} and the comparator mAb Orthoclone OKT3^{®} in the concentration range of 0.01 - 200 µg/ml corresponding to drug exposure levels *in vivo* typically attained during first-in-man infusions of therapeutic proteins. CD11b expression was determined by FACS. PTX3 release was measured either by colorimetric or by electrochemiluminescent custom sandwich ELISA.

After 2 hours of co-incubation with blood, all the tested drugs but Synagis^{®} induced an upregulation of CD11b greater than the 30% threshold for activation of neutrophils in both donors. Additionally, LPS, MabCampath^{®} and Orthoclone OKT3^{®} triggered dose-dependent PTX3 release in both ELISA assay platforms. Colorimetric and ECL ELISA PTX3 dose-response curves exhibited similar shape but side by side comparison of measured concentrations revealed a 100-fold difference between the two methods with values ranging from 0.1 - 0.6 ng/ml vs. 10 - 60 ng/ml respectively (Figure 1).

Blood levels of PTX3 reported in clinical trials and ex-vivo studies typically range from lower than 2 ng/ml in controls to 800 ng/ml for septic patients after 8 hours (Bottazzi B, Garlanda C, Salvatori G, Jeannin P, Manfredi A, Mantovani A.Pentraxins as a key component of innate immunity. Curr Opin Immunol (2006) 18: 10-15). These values correspond to the scale of PTX3 concentrations obtained with the ECL assay, thus favoring this assay as the method of choice for PTX3 measurement in the present study.

### TGN1412 like Antibody

Blood from 2 different individuals was treated for 24 hours with the positive control mAb MabCampath^{®}, or the negative control Ab Erbitux^{®} and the comparator TGN1412-like mAb in the concentration range 0.1-100 µg/ml corresponding to drug exposure levels *in vivo* typically attained during first-in-man infusions of therapeutic proteins. PTX3 release was measured electrochemiluminescent custom sandwich ELISA. Results are shown in Figure 8.

### Accuracy

To determine the most accurate PTX3 ELISA method, plasma derived from two human whole blood donations were spiked with increasing concentrations of recombinant human PTX3, covering the range 0 - 2500 pg/ml. Subsequently the signal for PTX3 was measured by ECL vs. colorimetric ELISA.

Upon measurement by ECL method, PTX3 recovery range across 10-2500 pg/ml spiked concentrations was 12-1365 pg/ml for one donor and 16-2104 pg/ml for the second one (Table 1).

When the very same samples were measured with the PTX3 colorimetric assay (Table 1), values ranged from 355-6464 pg/ml.

In conclusion, the recovery in the determination of PTX3 in heparinated human plasma was found to be more accurate using the ECL ELISA technology. Hence, this method was selected for further measurements of PTX3 in the present study.

### Matrix effect

To determine to what extent the matrix may interfere with the PTX3 detection, PTX3 standards were diluted in plasma generated from blood of two donors drawn on heparin or EDTA anticoagulants. Recovered PTX3 concentrations were compared to standard curve concentrations obtained in calibrator diluent.

With the ECL assay the overall percentage of PTX3 recovered across 10-2500 ng/ml was 85 ±13 % in heparinated plasma and above 4000% in EDTA plasma (Table 2).

The colorimetric assay is not suitable for measurement in heparinated blood as specified by the provider.

### Sensitivity

Samples from 25 healthy blood donors were analysed to determine the lower limit of detection (LLOD) and the lower limit of quantitation (LLOQ) (IUPAC Technical Report) Harmonized guidelines for single-laboratory validation of methods of analysis, Pure and Applied Chemistry (2002) 74:835-855 ) for the PTX3 endpoints.

The LLOD of PTX3 measurement was found to be of 0.065 ng/ml and the LLOQ of 0.466 ng/ml (Table 3). This is in accordance with the values provided by the supplier of the human PTX3 detection set antibodies, with a LLOD of 75 pg/ml for ELISA application.

As only values ranging from 2 - 1000 ng/ml PTX3 were considered relevant according to PTX3 analysis ex vivo, this assay qualifies very well with respect to its sensitivity to measure meaningful changes in PTX3 levels in human blood.

The flow-cytometric analysis of CD11b relies on the determination of a subset of cells relative to the whole set of cells in whole blood, for which a sensitivity of <1.0% is easily obtained with standard settings of flow-cytometric analysis. As only values above the cut-point of 30% CD11b+ neutrophils were considered relevant for the risk assessment, the sensitivity of the flow-cytometry analysis is considered appropriate.

### Dynamic range and kinetics of PTX3 response

With respect to the analysis of PTX3, the linear range of the calibration curve of the ECL PTX3 ELISA test typically ranges from 0.6 - 2500 ng/ml. This range covers the whole spectrum of PTX3 values observed in blood from patients ex vivo in clinical trials or across several indications (Bottazzi B, Garlanda C, Salvatori G, Jeannin P, Manfredi A, Mantovani A.Pentraxins as a key component of innate immunity. Curr Opin Immunol (2006) 18: 10-15; Latini R., Maggioni A.P., Peri G., Gonzini L, Lucci D, Mocarelli P, Vago L, Pasqualini F, Signorini S, Soldateschi D, Tarli L, Schweiger C, Fresco C, Cecere R, Tognoni G, Mantovani A; Lipid Assessment Trial Italian Network (LATIN) Investigators.Prognostic significance of the long pentraxin PTX3 in acute myocardial infarction. Circulation (2004) 110: 2349-2354; Muller B, Peri G, Doni A, Torri V, Landmann R, Bottazzi B, Mantovani A. Circulating levels of the long pentraxin PTX3 correlate with severity of infection in critically ill patients, Crit Care Med. (2001) 29:1404-1407) with a peak of PTX3 values in patient blood reported at 6-8h in the range 200 - 800 ng/ml during sepsis and endotoxic shock.

In our assay PTX3 release increased with drug concentrations and incubation time without ever reaching a plateau. Unlike cytokines and CD11b values which peak across the drug concentration applied. Although TNF-α and IFN-γ secretion intensify with the duration of drug incubation, after 2 hours the extent of cytokine secretion is sufficient to reach the 60 pg/ml threshold value in 90% of the donors tested.

After 2 hours of 0.01- 200 µg/ml MabCampath® challenge in whole blood, maximum values of 22 - 88 ng/ml PTX3 were obtained from 10 healthy donors (Figure 6C).

The dynamic range of PTX3 release in response to LPS 0.001 - 100 ng/ml was slightly broader, ranging from 22 -91 pg/ml for the 5 healthy individuals tested (Figure 6C).

In the flow-cytometry assay for determining the subset of CD11b+ neutrophils, indicative for activated neutrophils, the dynamic range lies in the interval of 1 - 100%. The values actually obtained in the neutrophil activation assessment typically ranged from 3 - 97% CD11b+ neutrophils (Figure 3, Table 5).

For the analysis of TNF-α, the linear part of the calibration curve of the MSD test typically ranges from 10-10 000 pg/ml, covering the whole set of TNF-α values reported in patient blood. ex vivo in clinical trials or post-marketing upon infusion of drugs triggering CRS (Moreau T, et al., Transient increase in symptoms associated with cytokine release in patients with multiple sclerosis. Brain. 119 (1996): 225-37; Moreau T, et al., Transient increase in symptoms associated with cytokine release in patients with multiple sclerosis. Brain. 119 (1996): 225-37; Winkler U, et al., Cytokine-release syndrome in patients with B-cell chronic lymphocytic leukemia and high lymphocyte counts after treatment with an anti-CD20 monoclonal antibody (Rituximab, IDEC-C2B8). Blood. 94 (1999): 2217-24).

In accordance with that, across the 4 hours of MabCampath® challenge peak values of 64-690 pg/ml TNF-α release were obtained in whole blood from 9 healthy donors. For one donor, cytokine levels at 2 hours were low, and TNF-α release did not reach the 60 pg/ml threshold (Figure 3, 4 and 5). Higher range was observed for IFN- γ peak values with concentrations of 12 - 1904 pg/ml (Figure 4).

### Specificity

The specificity of the whole blood assay was established on two levels: (i) donor-specific responses and (ii) drug-specific responses.

Donor-specificity was addressed by cytokine and CD11b analysis in the presence of mAb MabCampath^{®} and Orthoclone OKT3^{®}: Donor 2 was positive with respect to secretion of TNF-α and IFN-γ in response to MabCampath®. In contrast, donor 1 did not respond to Mabcampath^{®} with respect to induction of TNF-α and IFN-γ (Figure 3, Table 5). Donor differences were also observed in PTX3 measurements with baseline values of 35 ng/ml for donor 1 of the kinetic study versus 3-5 ng/ml for donors 3, 4 and 5 (Figure 4 and 5).

The assay did also confirm pronounced drug-specificity: mAb MabCampath^{®} did induce TNF-α and IFN-γ release in donor 3, whereas mAb Orthoclone OKT3^{®} did not (Table 5). The reverse trend was true for donor 1 who responded strongly to mAb Orthoclone OKT3^{®} by the induction of TNF-α and IFN-γ while secreted very low amounts of cytokines after mAb MabCampath^{®} challenge (Table 5).

### Coefficient of variance: Precision across concentrations and donors

To evaluate to what extent the coefficient of variance (CV) is dependent on the concentration of the respective antibody or on the choice of the donor, blood samples from 10 individuals were tested with mAb MabCampath® at 5 different concentrations, and the CV value obtained with the samples from 1 donor at the respective concentration of MabCampath® was compared with the CV values of the other 9 donors. Additionally, the mean and SD of all the CV values across concentrations were calculated and the minimal (CVmin) and maximal CV (CVmax) value per group determined (Table 4).

The analysis of the CV values for PTX3 revealed CVmin values of 1.6 - 2.5% and CVmax values of 13.4 - 23.5% (Table 4), with the mean CV values displaying a very low variance between the concentrations (5.1 - 9.8%).

A broader distribution in the CV values was found with the CD11b upregulation assay except for the lower end of the concentration curve (CVmin: 1.0 - 8.6%, CVmax : 4.7 - 42.0%, mean CV: 2.5 - 19.9%) (Table 4).

In conclusion, the PTX3 CV values varied moderately across donors and concentrations and did not show any correlation with the drug concentration. There were also no significant alterations in the CV values observed between samples from different donors.

### Threshold estimation

To evaluate a threshold above which a PTX3 release could be considered as significantly positive in the WBA, we determined the value of the mean + 2 SD for the PTX3 responses measured after 2 hours in the presence of the negative control mAb Synagis^{®}. Across all the concentrations and all the 10 donors of this study this cut-point value corresponds to 25 ng/ml (Table 6).

Consequently, based on the present results we propose that a 25 ± 5 ng/ml PTX3 concentration at 2 hours could be used as a threshold to distinguish positive and negative response in the WBA.

In such conditions, all donors 1-5 would be considered positive for their responses after 2 hour-incubation with mAb MabCampath® as well as with Orthoclone OKT3^{®}. Interestingly with mAb MabCampath® donor 1 would just reach the thresholds for both TNF-α (64 ±15 pg/ml) and PTX3 (22.7 ±3.5 ng/ml) release. Additionally, PTX3 response would align with cytokine secretion in qualifying donor 4 as positive with mAb MabCampath®, while CD11b upregulation was below the 30% threshold (Table 5). Moreover, donor 3 which as expected, did not respond to Synagis^{®} in terms of cytokine secretion, would also appear negative with regards to PTX3 release (13.99 ±0.6 ng/ml). In contrast, donor 1 of the kinetic study would then be considered positive for PTX3 independent of the drug exposure, simply due to its high PTX3 baseline levels (36.45 ±2.01 ng/ml with Synagis^{®} 0.5hr).

In summary, samples attaining PTX3 levels >25 ng/ml PTX3 in plasma after 2 hours could be considered "positive" for the risk of clinically relevant cytokine release.

In comparison to the CD11b assay involving subsets of peripheral blood mononuclear cells (PBMC) to evaluate the degree of neutrophil activation, PTX3 measurements based on the ECL ELISA custom technology appear to have the following advantages:
- heparinated plasma is the relevant compartment *in vivo*
- dilution steps and addition of Ficoll (necessary for PBMC preparation) are avoided
- presence of full set of cells, including neutrophils and eosinophils in the whole blood
- single (ECL) instead of double (ELISA and FACS) assay format
- assessment is done within the relevant time frame of 2 hrs, when typical symptoms of cytokine release syndrome are observed *in vivo*

### Tables

**Table 1: Accuracy.** Enhanced Chemiluminescence (ECL) ELISA (table 1 a) and colorimetric assay (table 1b) were compared as detection method for PTX3. The measurements were done with plasma from two donors comprising either heparin or EDTA anticoagulants. Plasma derived from two human whole blood donations were spiked with increasing concentrations of recombinant human PTX3, covering the range 0 - 2500 pg/ml. #: mean from triplicates, n.e.: not evaluable

**Table 1a)**

| **Spiked PTX3 concentration (ng/ml)** | **Recovered PTX3 in plasma (ng/ml), via ECL ELISA assay** | | | | | | | |
|---|---|---|---|---|---|---|---|---|
| | Heparinated blood | | | | EDTA blood | | | |
| | Donor α | | Donor β | | Donor α | | Donor β | |
| | Mean^{#} | SD | Mean^{#} | SD | Mean^{#} | SD | Mean^{#} 4 | SD |
| 2500 | 1365.37 | 16.66 | 2104.44 | 266.64 | 7737.86 | 245.14 | 9133.14 | 260.65 |
| 625 | 382.18 | 19.49 | 636.31 | 36.54 | 7513.31 | 167.49 | 8897.38 | 99.69 |
| 156 | 88.78 | 1.17 | 186.01 | 8.07 | 6749.16 | 109.70 | 8117.68 | 77.07 |
| 39 | 25.45 | 0.67 | 54.16 | 6.84 | 3669.54 | 125.20 | 5739.99 | 216.99 |
| 9.8 | 12.41 | 3.27 | 16.66 | 1.27 | 1144.22 | 91.47 | 1058.49 | 57.79 |
| 2.4 | 11.25 | 16.66 | 7.28 | 266.64 | 281.20 | 245.14 | 168.54 | 260.65 |

**Table 1b)**

| **Spiked PTX3 concentration (ng/ml)** | **Recovered PTX3 in plasma (ng/ml), via colorimetric assay** | | | | | | | |
|---|---|---|---|---|---|---|---|---|
| | Heparinated blood | | | | EDTA blood | | | |
| | Donor α | | Donor β | | Donor α | | Donor β | |
| | Mean^{#} | SD | Mean^{#} | SD | Mean^{#} | SD | Mean^{#} | SD |
| 2500 | n.e. | n.e. | n.e. | n.e. | n.e. | n.e. | n.e. | n.e. |
| 625 | n.e. | n.e. | n.e. | n.e. | n.e. | n.e. | n.e. | n.e. |
| 156 | 1.69 | 0.04 | 2.71 | 0.29 | n.e. | n.e. | n.e. | n.e. |
| 39 | 0.63 | 0.02 | 0.97 | 0.06 | n.e. | n.e. | n.e. | n.e. |
| 9.8 | 0.36 | 0.05 | 0.40 | 0.01 | n.e. | n.e. | n.e. | n.e. |
| 2.4 | 0.17 | 0.05 | 0.25 | 0.09 | 2.62 | 0.02 | 2.13 | 0.03 |

**Table 2: Matrix effect.** Enhanced Chemiluminescence (ECL) ELISA (table 2a) and colorimetric assay (table 2b) were compared as detection method for PTX3. The measurements were taken in plasma generated from blood of two donors drawn on heparin or EDTA anticoagulants.

**Table 2a)**

| **Standards** (ng/ml) | **Recovered PTX3 (%) in pure plasma, ECL assay** | | | | | | | |
|---|---|---|---|---|---|---|---|---|
| | Heparinated plasma | | | | EDTA plasma | | | |
| | Donor α | Donor β | **Mean** | **SD** | Donor α | Donor β | **Mean** | **SD** |
| 2500 | 55 | 84 | 69 | 21 | 310 | 365 | 337 | 39 |
| 625 | 61 | 102 | 81 | 29 | 1202 | 1424 | 1313 | 157 |
| 156 | 57 | 119 | 88 | 44 | 4326 | 5204 | 4765 | 620 |
| 39 | 65 | 139 | 102 | 52 | 9409 | 14718 | 12063 | 3754 |
| 9.8 | 127 | 170 | 148 | 31 | 11676 | 10801 | 11238 | 619 |
| 2.4 | 469 | 304 | 386 | 117 | 11717 | 7022 | 9370 | 3319 |
| 0.6 | 664 | 890 | 777 | 160 | 16366 | 7885 | 12126 | 5997 |

**Table 2b)**

| **Standards** (ng/ml) | **Recovered PTX3 (%) in pure plasma, colorimetric assay** | | | | | | | |
|---|---|---|---|---|---|---|---|---|
| | Heparinated plasma | | | | EDTA plasma | | | |
| | Donor α | Donor β | **Mean** | **SD** | Donor α | Donor β | **Mean** | **SD** |
| 2500 | n.e. | n.e. | n.e. | n.e. | n.e. | n.e. | n.e. | n.e. |
| 625 | n.e. | n.e. | n.e. | n.e. | n.e. | n.e. | n.e. | n.e. |
| 156 | 1.08 | 1.73 | 1.40 | 0.11 | n.e. | n.e. | n.e. | n.e. |
| 39 | 1.62 | 2.47 | 2.04 | 0.10 | n.e. | n.e. | n.e. | n.e. |
| 9.8 | 3.62 | 4.05 | 3.84 | 0.29 | n.e. | n.e. | n.e. | n.e. |
| 2.4 | 7.17 | 10.25 | 8.71 | 1.05 | 109 | 89 | 98.90 | 5.99 |
| 0.6 | 24.67 | 22.00 | 23.33 | 2.47 | 234 | 127 | 180.08 | 3.24 |

**Table 3: Sensitivity of cytokine determination; Lower limit of detection and lower limit of quantification**

| **Analyte** | **Lower Limit of Detection** (pg/ml) | **Lower Limit of Quantification** (pg/ml) |
|---|---|---|
| | **LLOD** | **LLOQ** |
| **PTX3** | **65** | **466** |
| **TNF**-α | 0.007 | 0.057 |
| **IFN**-γ | 0.041 | 0.327 |

**Table 4: Coefficient of variance (CV) for analysis of PTX3 (table 4a) and CD11b (table 4b): Precision across donors and concentrations (0.1 to 200 µg/ml). (SD = Standard deviation)**

**Table 4a)**

| **mAb** (µg/ml) | **PTX3** | | | |
|---|---|---|---|---|
| | Mean CV (%) | SD CV (%) | CVmin (%) | CVmax (%) |
| 200 | 5.10 | 4.18 | 2.04 | 15.65 |
| 100 | 8.04 | 5.69 | 1.65 | 13.45 |
| 10 | 7.80 | 4.67 | 2.32 | 16.02 |
| 1 | 9.86 | 4.34 | 2.56 | 15.47 |
| 0.1 | 8.27 | 6.10 | 1.96 | 23.59 |

**Table 4b)**

| **mAb** (µg/ml) | **CD11b** | | | |
|---|---|---|---|---|
| | Mean CV (%) | SD CV (%) | CVmin (%) | CV max (%) |
| 200 | 5.09 | 5.71 | 1.08 | 14.73 |
| 100 | 2.53 | 1.29 | 1.41 | 4.73 |
| 10 | 2.59 | 2.33 | 1.03 | 6.71 |
| 1 | 8.42 | 6.55 | 1.05 | 18.99 |
| 0.1 | 19.97 | 13.50 | 8.61 | 42.06 |

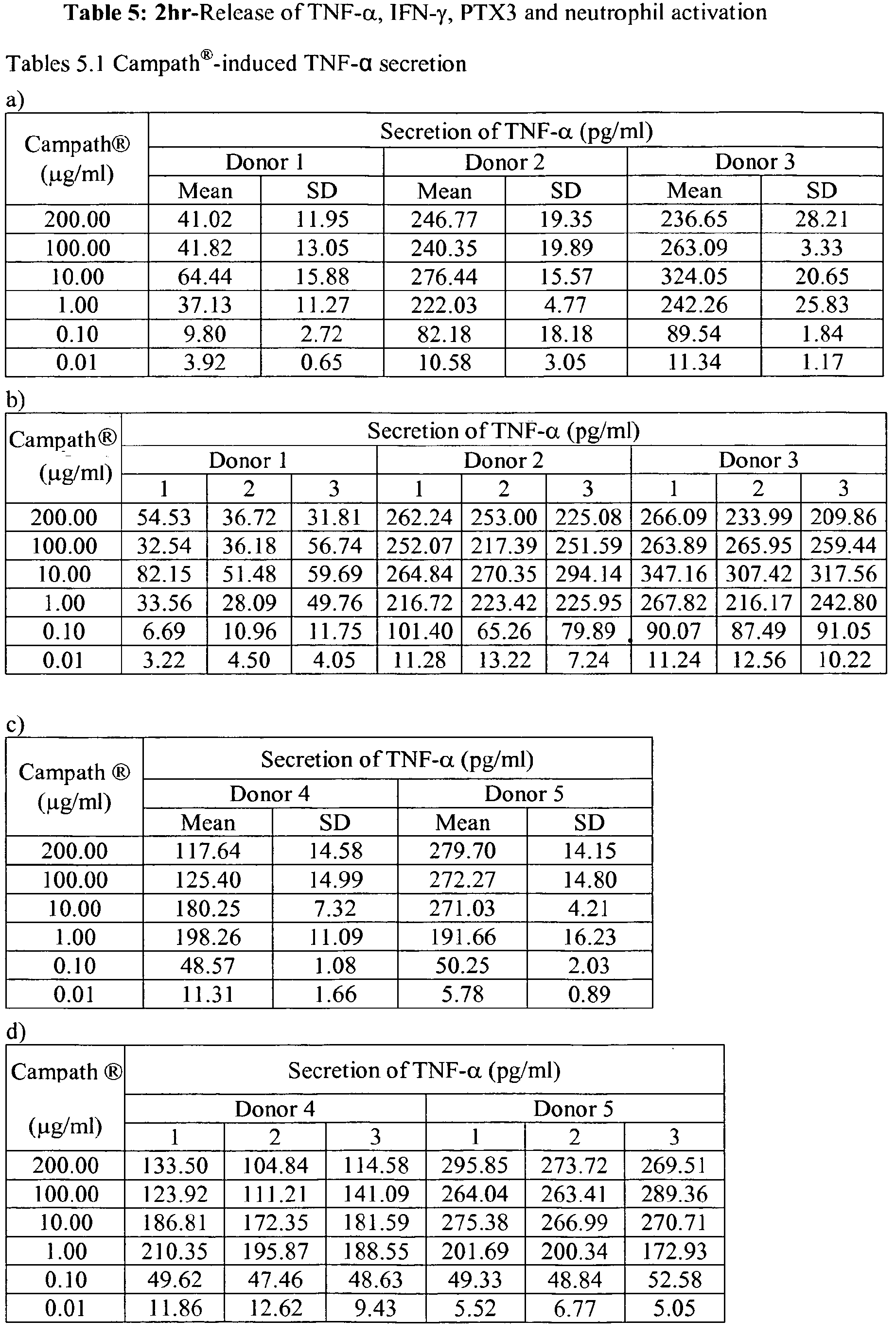

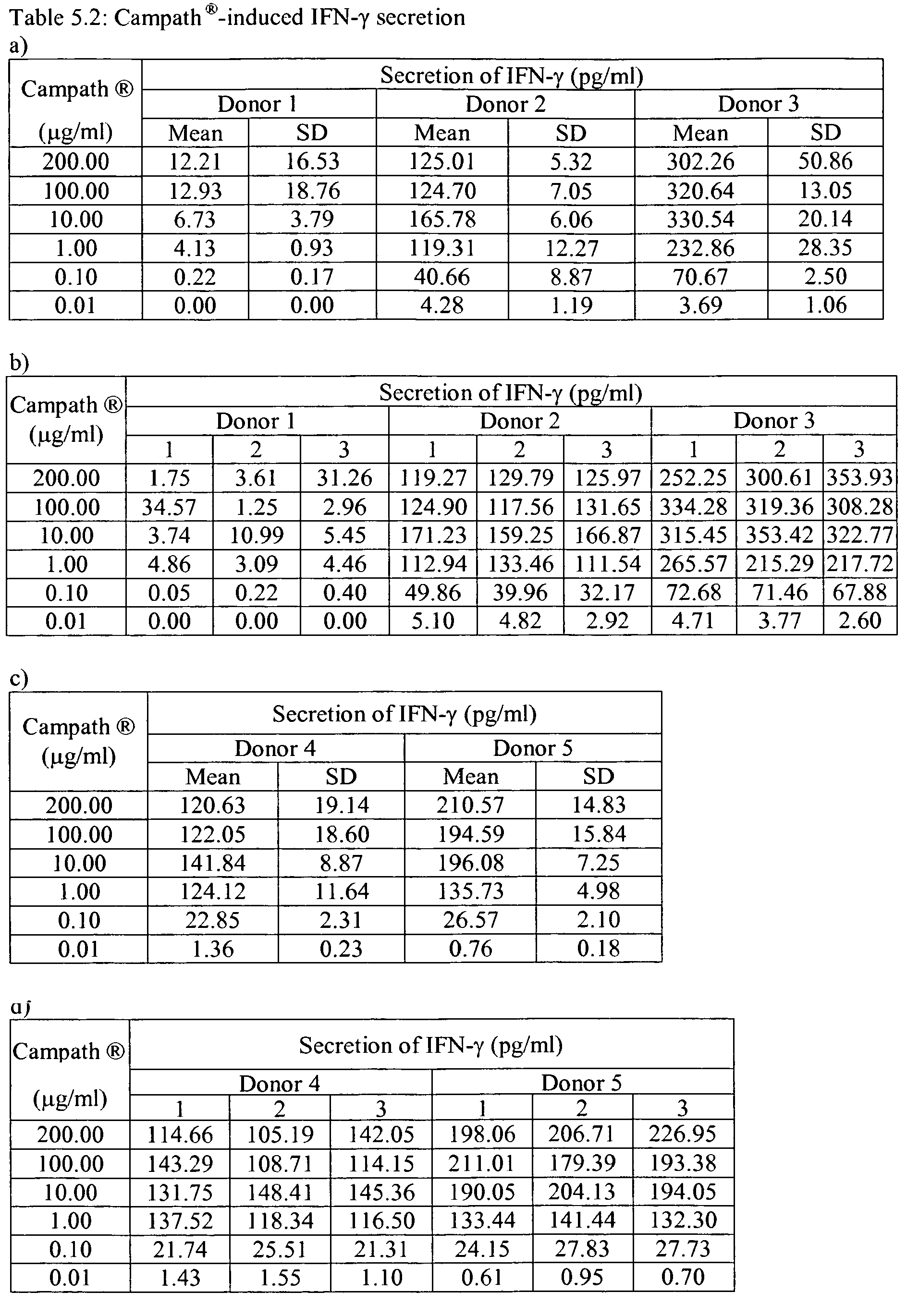

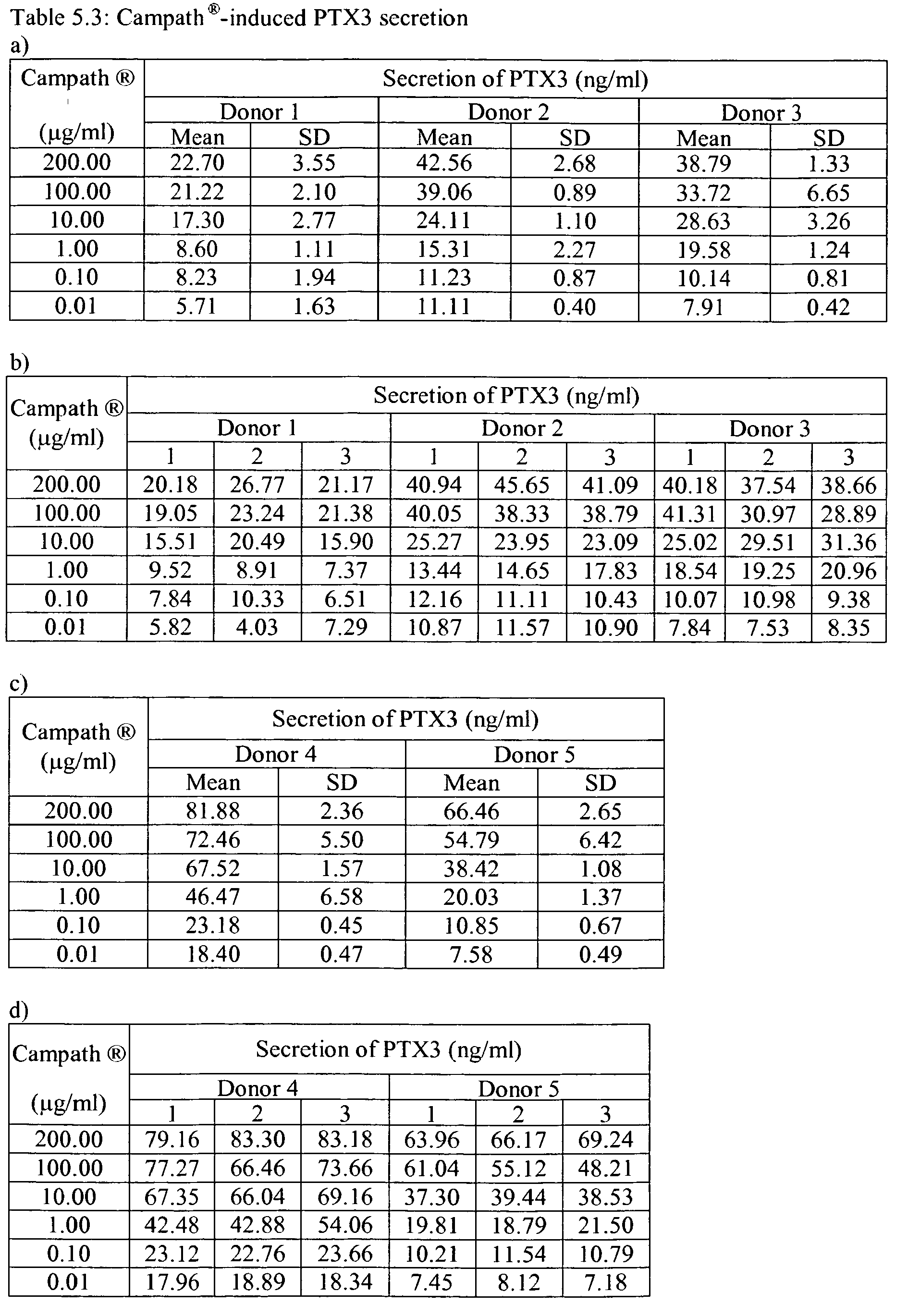

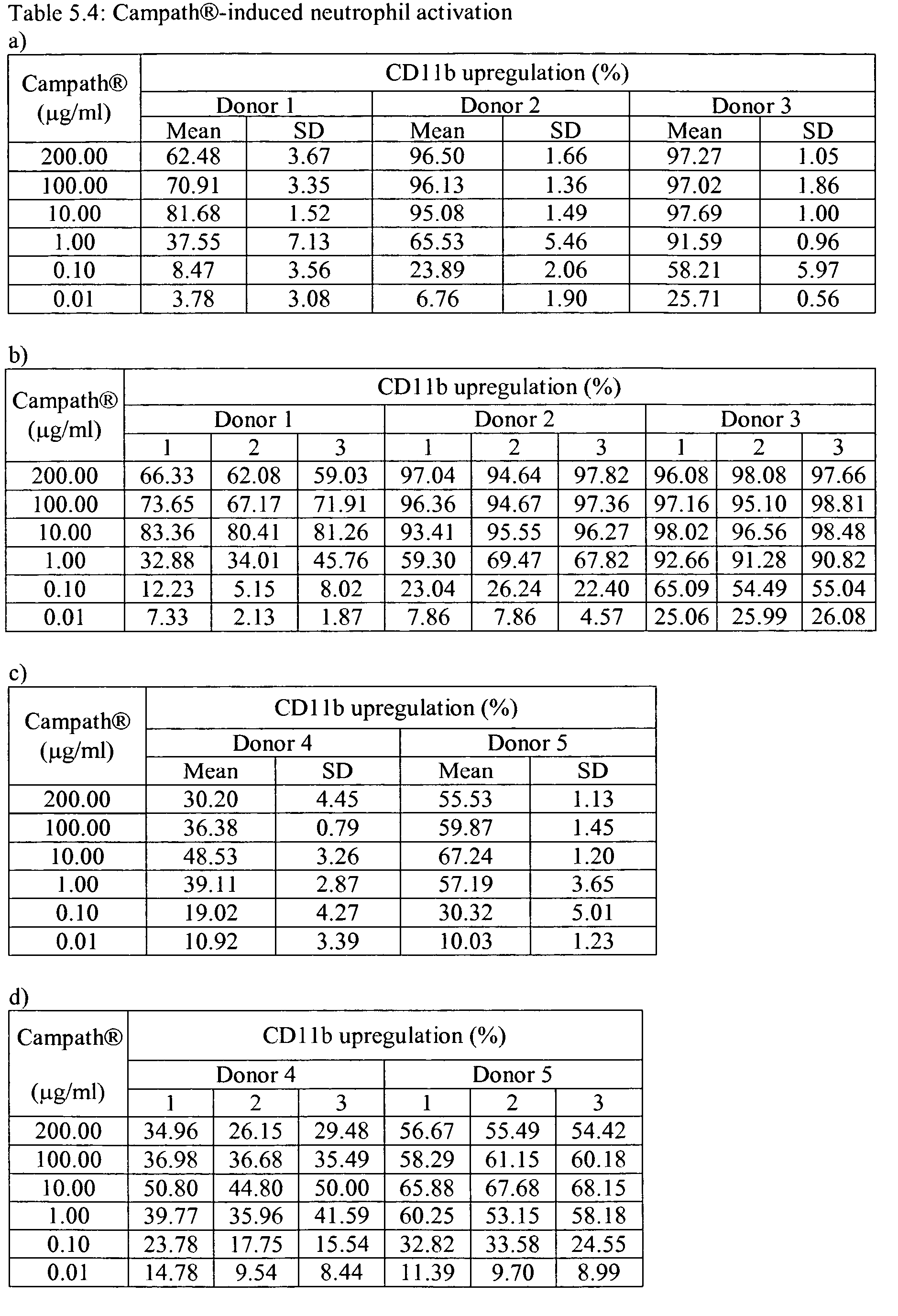

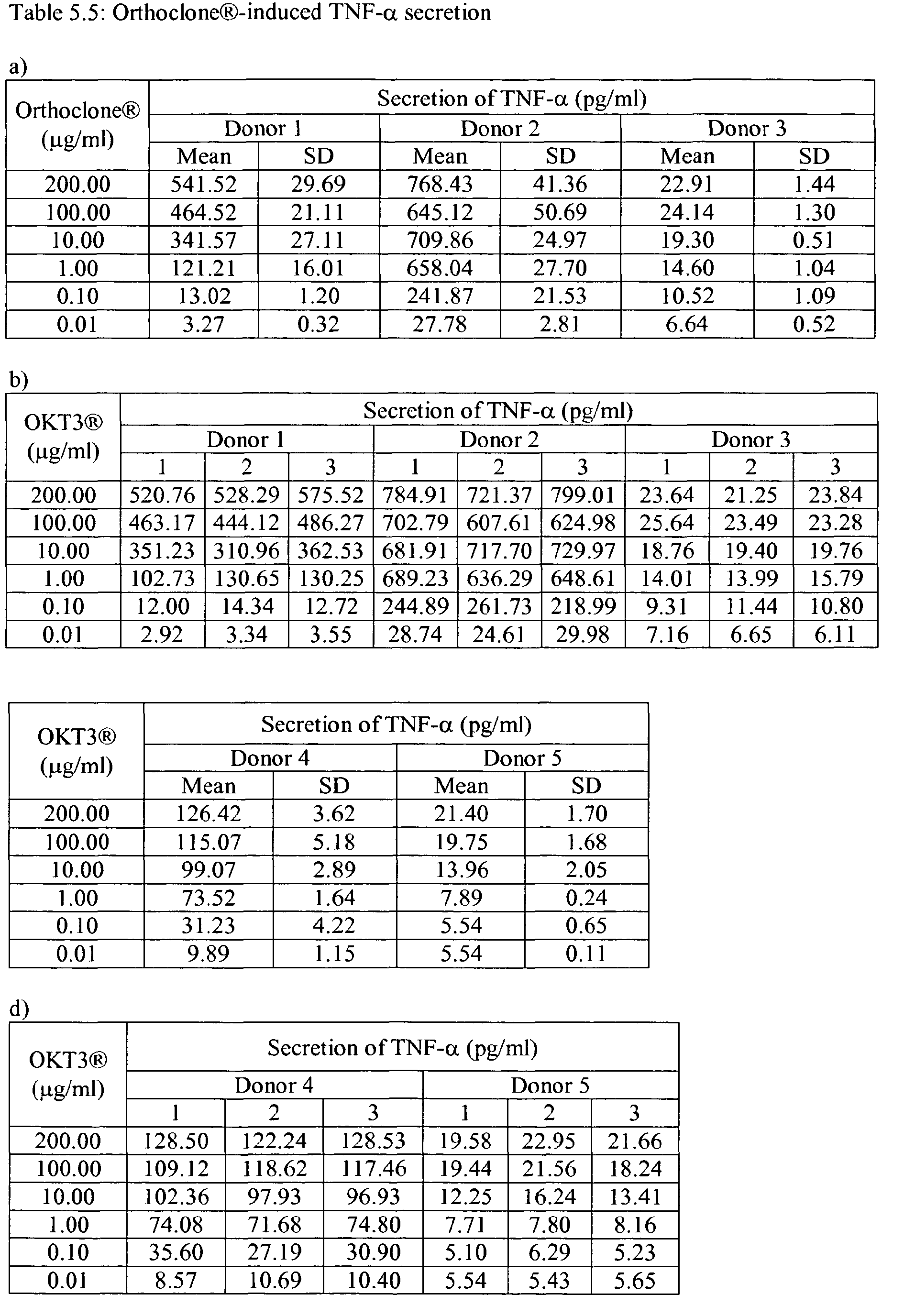

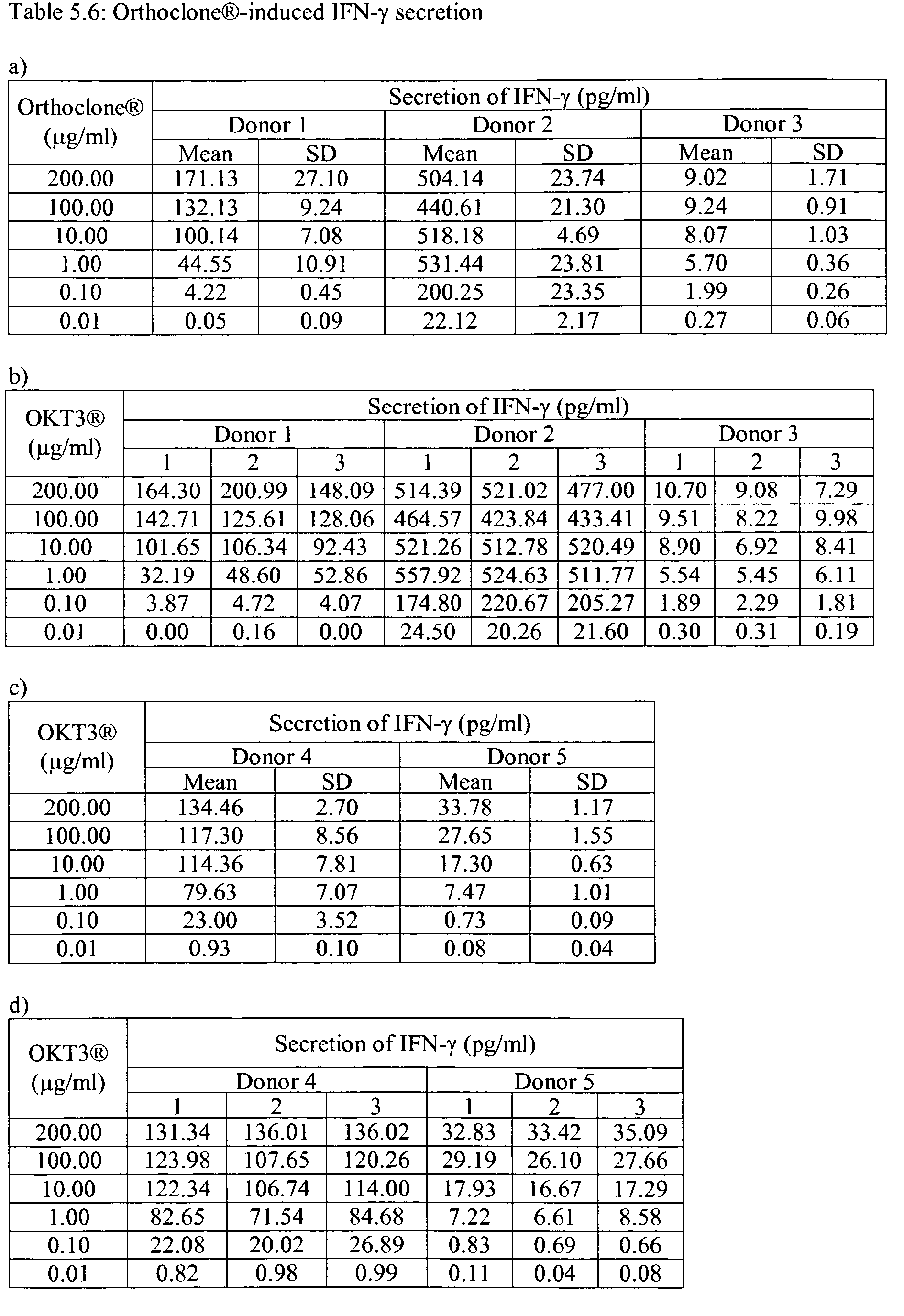

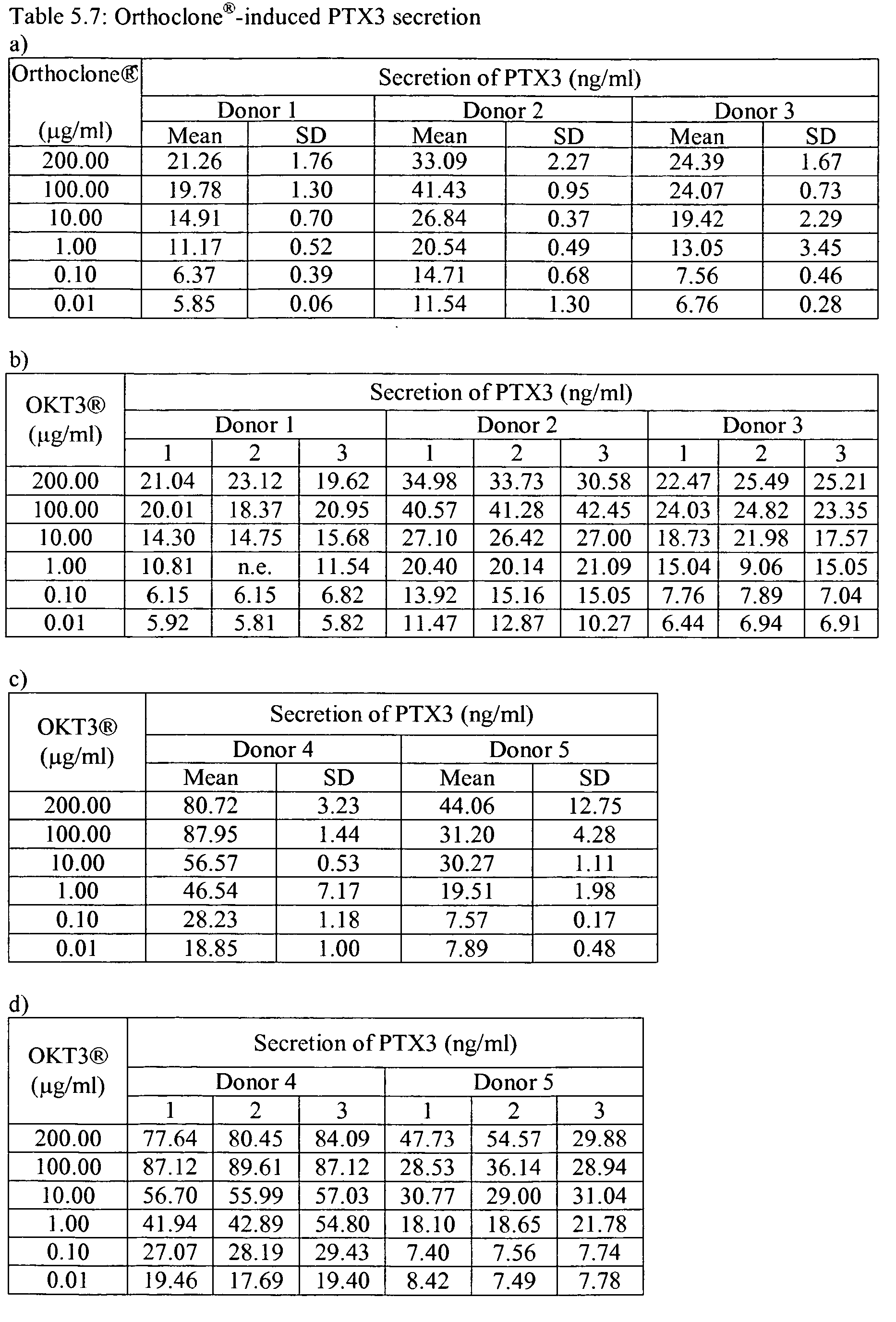

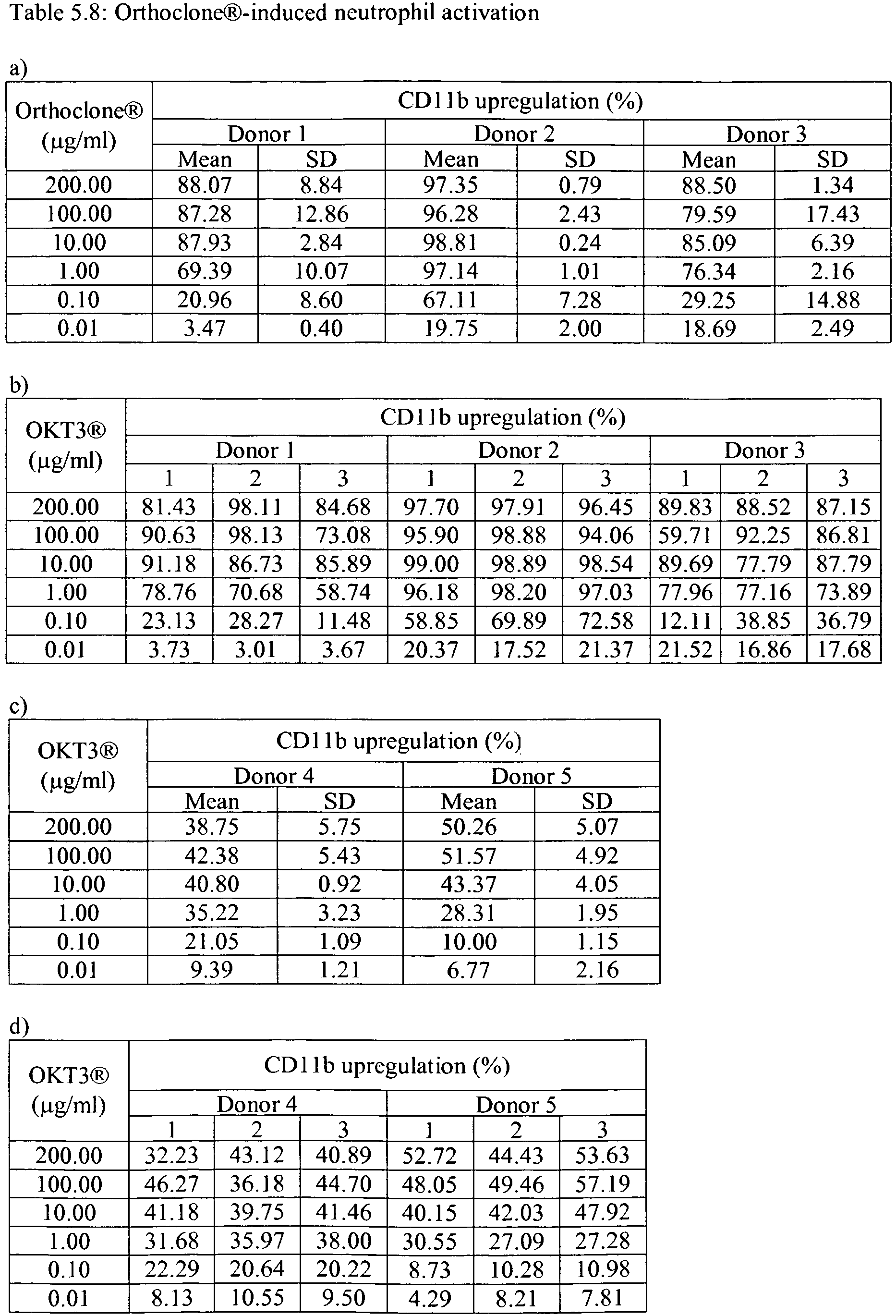

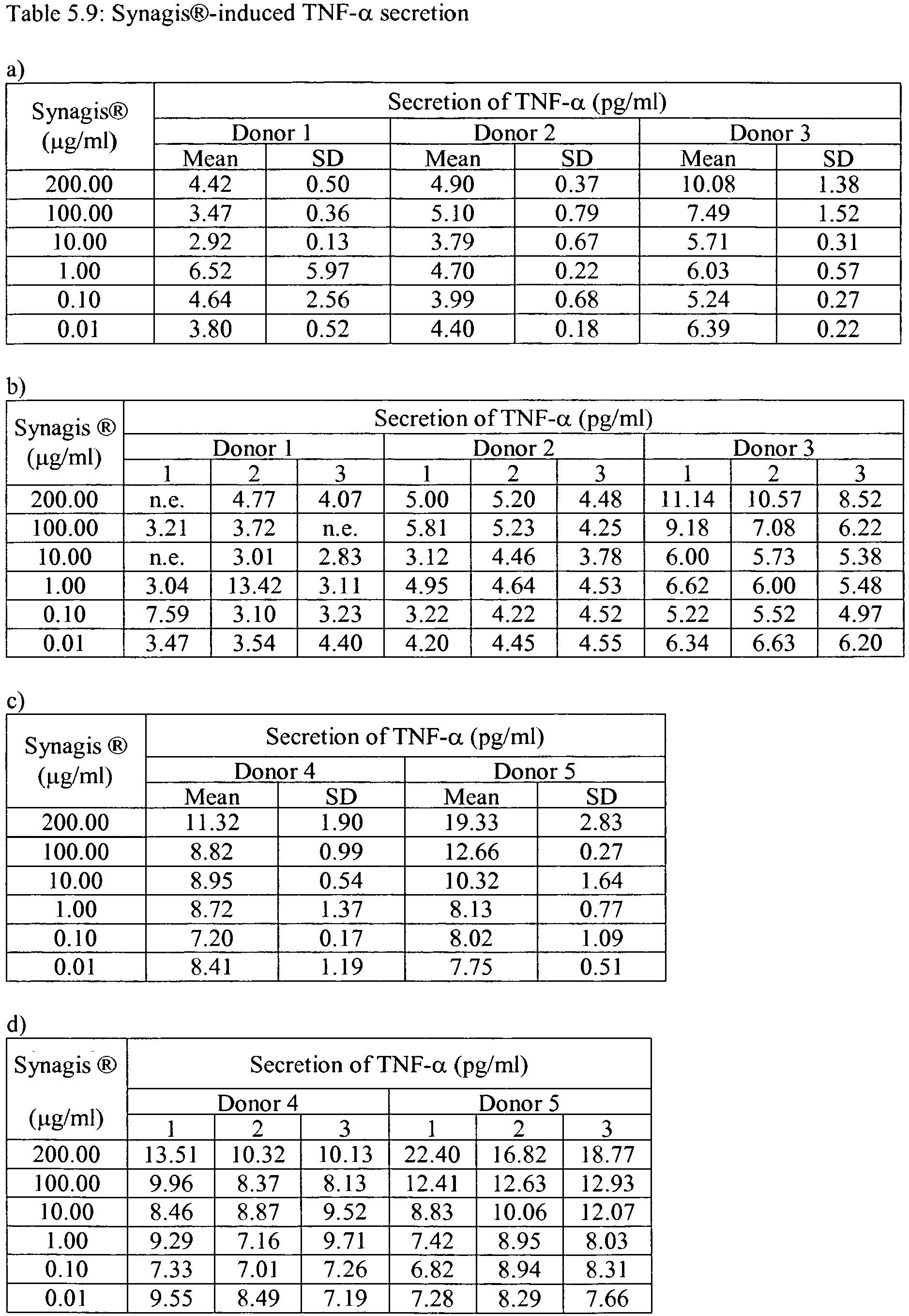

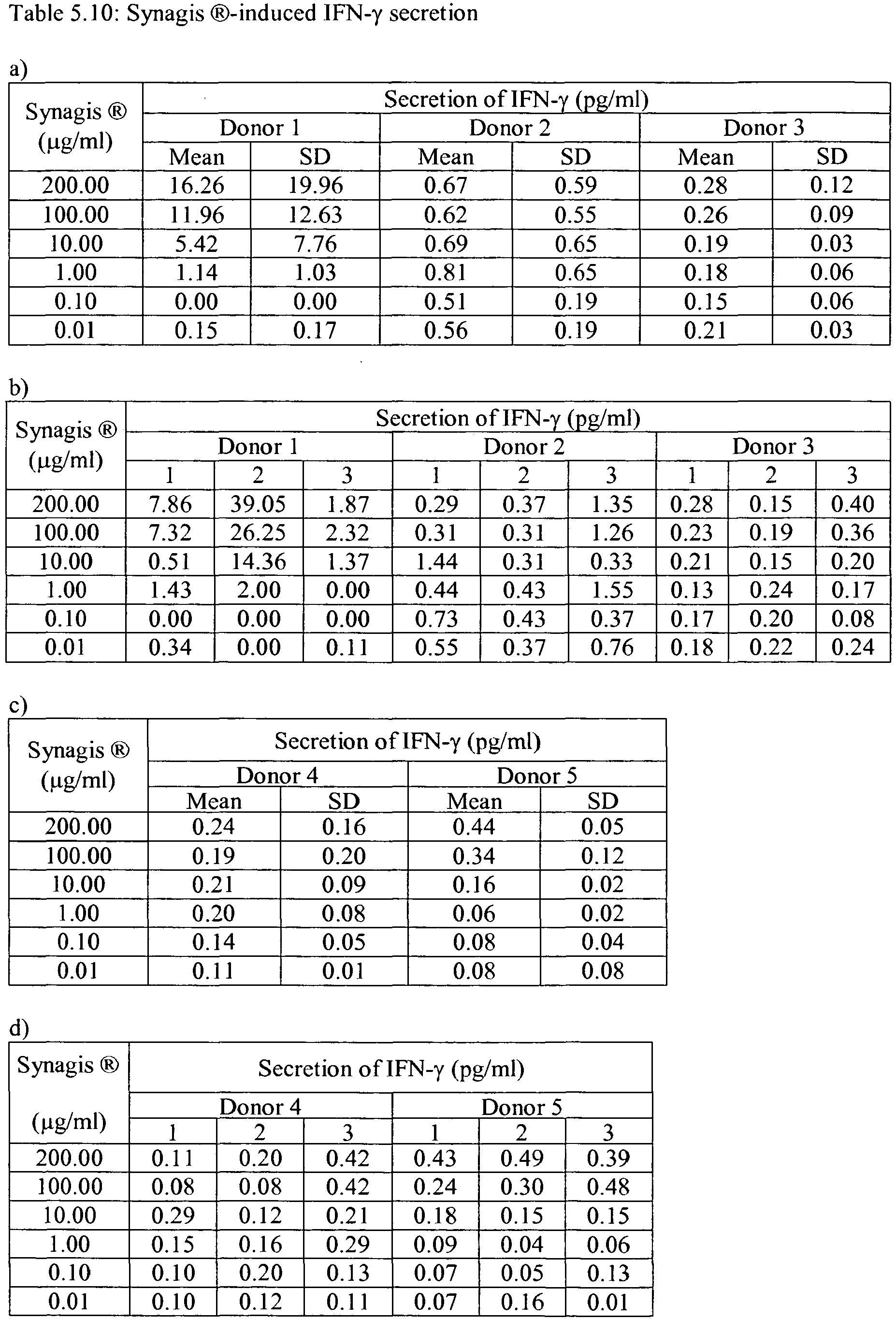

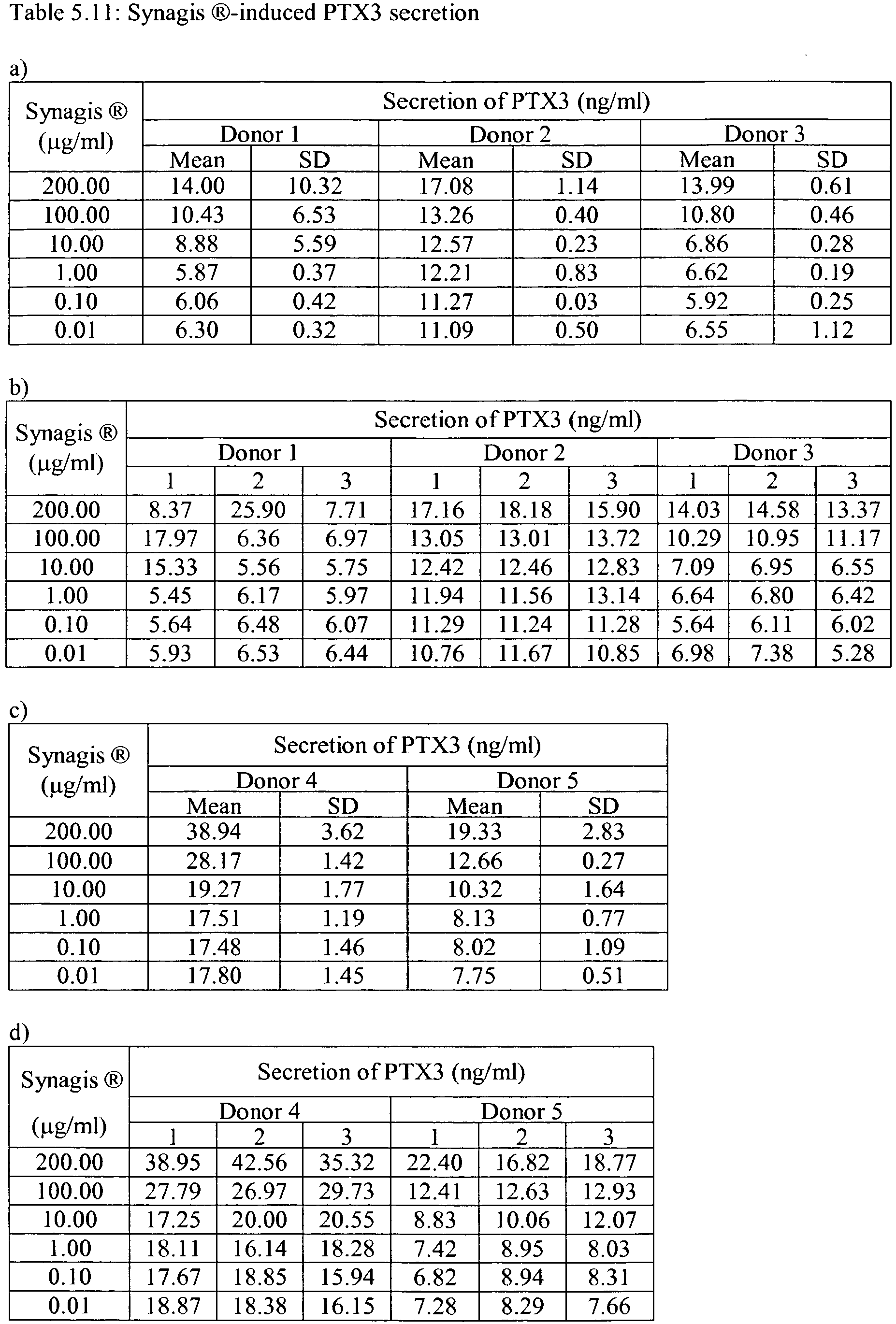

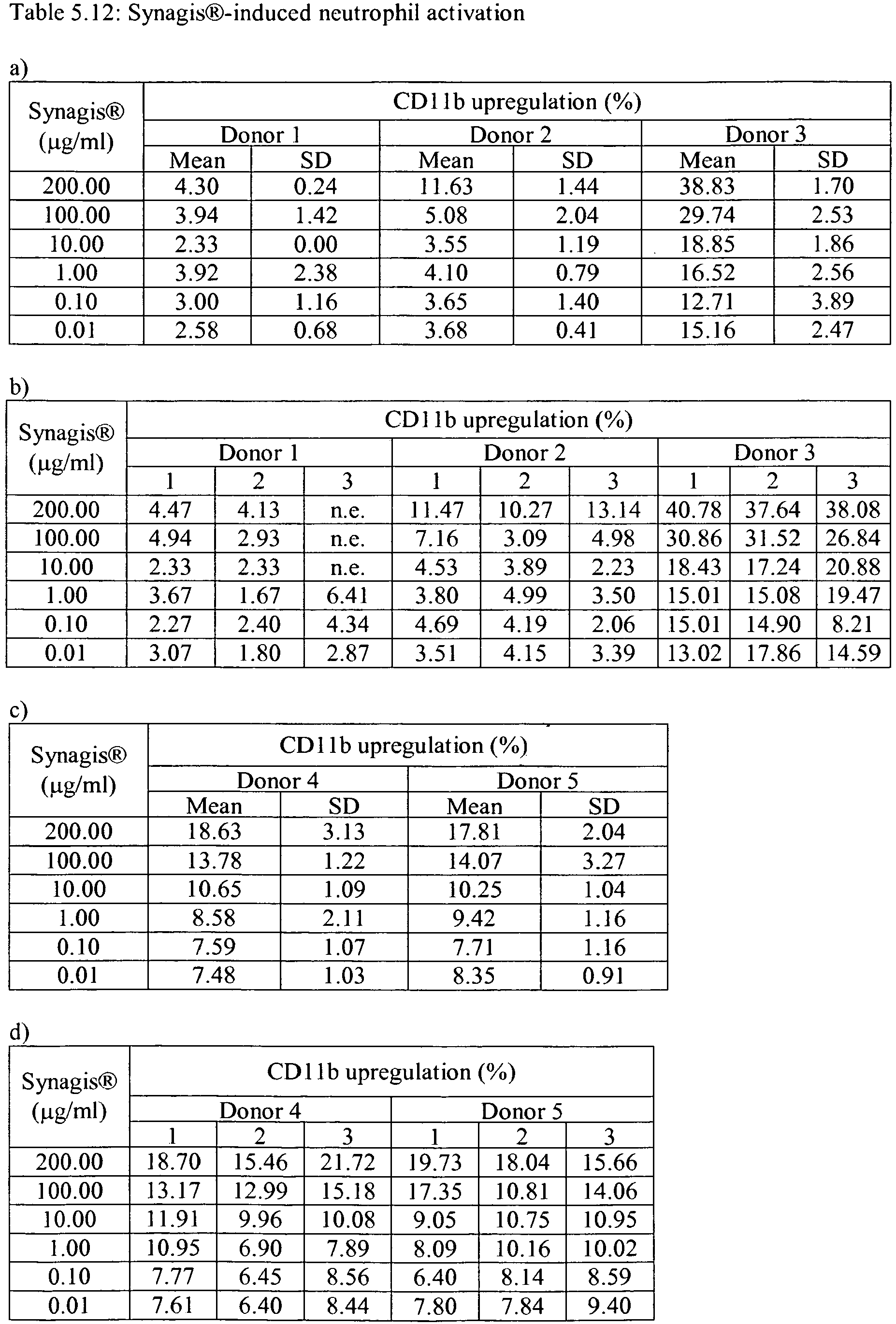

**Table 6: PTX3 Threshold estimation**

| Synagis® (µg/ml) | PTX3 (ng/ml) | | | | | | | | | | | |
|---|---|---|---|---|---|---|---|---|---|---|---|---|
| | Donor | | | | | | | | | | | |
| | 1 | 2 | 3 | 4 | 5 | 6 | 7 | 8 | 9 | 10 | Mean | SD |
| 200.00 | 14.00 | 17.08 | 13.99 | 38.94 | 19.33 | 57.30 | 30.58 | 14.15 | 16.37 | 14.08 | 23.58 | 14.52 |
| 100.00 | 10.43 | 13.26 | 10.80 | 28.17 | 12.66 | 50.59 | 18.84 | 12.08 | 14.49 | 8.96 | 18.03 | 12.72 |
| 10.00 | 8.88 | 12.57 | 6.86 | 19.27 | 10.32 | 46.38 | 14.32 | 10.14 | 7.99 | 6.73 | 14.34 | 11.89 |
| 1.00 | 5.87 | 12.21 | 6.62 | 17.51 | 8.13 | 44.83 | 13.03 | 10.16 | 7.15 | 6.53 | 13.20 | 11.71 |
| 0.10 | 6.06 | 11.27 | 5.92 | 17.48 | 8.02 | 44.97 | 12.38 | 9.42 | 7.29 | 5.63 | 12.84 | 11.87 |
| 0.01 | 6.30 | 11.09 | 6.55 | 17.80 | 7.75 | 44.16 | 12.17 | 10.18 | 7.20 | 6.07 | 12.93 | 11.56 |
| All concentrations mean and SD | | | | | | | | | | | 16.40 | 4.51 |
| Mean + 2 SD | | | | | | | | | | | 25.42 | |

## Claims

1. An *in vitro* method for determining the risk of a compound to induce a cytokine release syndrome in a human being comprising the steps of:
a) providing a whole blood sample, wherein said blood was exposed at least 30 minutes to said compound,
b) measuring the level of pentraxin-3 (PTX-3) in said sample, and
c) comparing the measured level of pentraxin-3 with a control, wherein a control is the pentraxin-3 level of an unexposed whole blood sample, wherein a significantly increased level of pentraxin-3 as compared to the control indicates a risk of said compound to induce acytoline release syndrome.

2. The method according to claim 1 wherein the PTX-3 level is measured in the plasma or serum obtained from the whole blood sample.

3. The method according to claim 1 or 2, wherein the whole blood comprises an anticoagulant.

4. The method according to claim 2, wherein the plasma comprises an anticoagulant.

5. The method according to claim 3 or 4, wherein the anticoagulant is heparin.

6. The method according to any one of claims 1 to 5, wherein the incubation of whole blood sample with a compound of interest has started within 4 hours after the collection of the blood sample.

7. The method according to any one of claims 1 to 6, wherein the whole blood sample is incubated with the compound of interest for 30 minutes to 24hours.

8. The method according to any one of the claims 1 to 7, wherein the level of pentraxin-3 is measured with Enhanced Chemiluminescence ELISA.

## Patentansprüche

1. *In vitro*-Verfahren zum Bestimmen des Risikos einer Verbindung, ein Cytokine-Release-Syndrom in einem Menschen auszulösen, umfassend die Schritte:
a) Bereitstellen einer Vollblutprobe, wobei das Blut der Verbindung mindestens 30 Minuten ausgesetzt war,
b) Messen des Pentraxin-3 (PTX-3)-Spiegels in der Probe, und
c) Vergleichen des gemessenen Pentraxin-3-Spiegels mit einer Kontrolle, wobei eine Kontrolle der Pentraxin-3-Spiegel einer nicht-ausgesetzten Vollblutprobe ist,
wobei ein signifikant erhöhter Pentraxin-3-Spiegel im Vergleich zu der Kontrolle ein Risiko der Verbindung anzeigt, ein Cytokine-Release-Syndrom auszulösen.

2. Verfahren gemäß Anspruch 1, wobei der PTX-3-Spiegel in dem aus der Vollblutprobe erhaltenen Plasma oder Serum gemessen wird.

3. Verfahren gemäß Anspruch 1 oder 2, wobei das Vollblut ein Antikoagulans umfasst.

4. Verfahren gemäß Anspruch 2, wobei das Plasma ein Antikoagulans umfasst.

5. Verfahren gemäß Anspruch 3 oder 4, wobei das Antikoagulans Heparin ist.

6. Verfahren gemäß einem der Ansprüche 1 bis 5, wobei die Inkubation der Vollblutprobe mit einer Verbindung von Interesse innerhalb von 4 Stunden nach der Entnahme der Blutprobe gestartet wird.

7. Verfahren gemäß einem der Ansprüche 1 bis 6, wobei die Vollblutprobe 30 Minuten bis 24 Stunden mit der Verbindung von Interesse inkubiert wird.

8. Verfahren gemäß einem der Ansprüche 1 bis 7, wobei der Pentraxin-3-Spiegel mit einem Enhanced Chemiluminescence-ELISA gemessen wird.

## Revendications

1. Procédé *in vitro* pour déterminer le risque qu'un composé induise un syndrome de libération de cytokines chez un être humain, comprenant les étapes consistant à :
a) fournir un échantillon de sang entier, dans lequel ledit sang a été exposé pendant au moins 30 minutes audit composé,
b) à mesurer le taux de pentraxine-3 (PTX-3) dans ledit échantillon, et
c) à comparer le taux mesuré de pentraxine-3 avec un témoin,
dans lequel un témoin est le taux de pentraxine-3 d'un échantillon de sang entier non exposé,
dans lequel un taux significativement accru de pentraxine-3, comparativement au témoin, indique un risque que ledit composé induise un syndrome de libération de cytokines.

2. Procédé suivant la revendication 1, dans lequel le taux de PTX-3 est mesuré dans le plasma ou le sérum obtenu à partir de l'échantillon de sang entier.

3. Procédé suivant la revendication 1 ou 2, dans lequel le sang entier comprend un anticoagulant.

4. Procédé suivant la revendication 2, dans lequel le plasma comprend un anticoagulant.

5. Procédé suivant la revendication 3 ou 4, dans lequel l'anticoagulant est l'héparine.

6. Procédé suivant l'une quelconque des revendications 1 à 5, dans lequel l'incubation de l'échantillon de sang entier avec un composé intéressant a débuté dans les limites de 4 heures après le prélèvement de l'échantillon de sang.

7. Procédé suivant l'une quelconque des revendications 1 à 6, dans lequel l'échantillon de sang entier est mis en incubation avec le composé intéressant pendant un temps de 30 minutes à 24 heures.

8. Procédé suivant l'une quelconque des revendications 1 à 7, dans lequel le taux de pentraxine-3 est mesuré par ELISA a chimiluminescence amplifiée.


### SEQUENCE LISTING

<110> F. Hoffmann-La Roche AG
<120> Drug safety assay
<130> 25129
<150> EP 08158724.8
   <151> 2008-06-20
<160> 1
<170> PatentIn version 3.5
<210> 1
   <211> 381
   <212> PRT
   <213> Homo sapiens
<300>
   <308> NP_002843
   <309> 2008-04-28
   <313> (1) .. (381)
<400> 1
   Met His Leu Leu Ala Ile Leu Phe Cys Ala Leu Trp Ser Ala Val Leu
   1               5                   10                  15
   Ala Glu Asn Ser Asp Asp Tyr Asp Leu Met Tyr Val Asn Leu Asp Asn
               20                  25                  30
   Glu Ile Asp Asn Gly Leu His Pro Thr Glu Asp Pro Thr Pro Cys Ala
           35                  40                  45
   Cys Gly Gln Glu His Ser Glu Trp Asp Lys Leu Phe Ile Met Leu Glu
       50                  55                  60
   Asn Ser Gln Met Arg Glu Arg Met Leu Leu Gln Ala Thr Asp Asp Val
   65                  70                  75                  80
   Leu Arg Gly Glu Leu Gln Arg Leu Arg Glu Glu Leu Gly Arg Leu Ala
                   85                  90                  95
   Glu Ser Leu Ala Arg Pro Cys Ala Pro Gly Ala Pro Ala Glu Ala Arg
               100                 105                 110
   Leu Thr Ser Ala Leu Asp Glu Leu Leu Gln Ala Thr Arg Asp Ala Gly
           115                 120                 125
   Arg Arg Leu Ala Arg Met Glu Gly Ala Glu Ala Gln Arg Pro Glu Glu
       130                 135                 140
   Ala Gly Arg Ala Leu Ala Ala Val Leu Glu Glu Leu Arg Gln Thr Arg
   145                 150                 155                 160
   Ala Asp Leu His Ala Val Gln Gly Trp Ala Ala Arg Ser Trp Leu Pro
                   165                 170                 175
   Ala Gly Cys Glu Thr Ala Ile Leu Phe Pro Met Arg Ser Lys Lys Ile
               180                 185                 190
   Phe Gly Ser Val His Pro Val Arg Pro Met Arg Leu Glu Ser Phe Ser
           195                 200                 205
   Ala Cys Ile Trp Val Lys Ala Thr Asp Val Leu Asn Lys Thr Ile Leu
       210                 215                 220
   Phe Ser Tyr Gly Thr Lys Arg Asn Pro Tyr Glu Ile Gln Leu Tyr Leu
   225                 230                 235                 240
   Ser Tyr Gln Ser Ile Val Phe Val Val Gly Gly Glu Glu Asn Lys Leu
                   245                 250                 255
   Val Ala Glu Ala Met Val Ser Leu Gly Arg Trp Thr His Leu Cys Gly
               260                 265                 270
   Thr Trp Asn Ser Glu Glu Gly Leu Thr Ser Leu Trp Val Asn Gly Glu
           275                 280                 285
   Leu Ala Ala Thr Thr Val Glu Met Ala Thr Gly His Ile Val Pro Glu
       290                 295                 300
   Gly Gly Ile Leu Gln Ile Gly Gln Glu Lys Asn Gly Cys Cys Val Gly
   305                 310                 315                 320
   Gly Gly Phe Asp Glu Thr Leu Ala Phe Ser Gly Arg Leu Thr Gly Phe
                   325                 330                 335
   Asn Ile Trp Asp Ser Val Leu Ser Asn Glu Glu Ile Arg Glu Thr Gly
               340                 345                 350
   Gly Ala Glu Ser Cys His Ile Arg Gly Asn Ile Val Gly Trp Gly Val
           355                 360                 365
   Thr Glu Ile Gln Pro His Gly Gly Ala Gln Tyr Val Ser
       370                 375                 380